# EUROPEAN PATENT SPECIFICATION

(11) **EP 1 706 485 B1**
(45) Date of publication and mention of the grant of the patent: **26.09.2007**
(21) Application number: 05706999.9
(22) Date of filing: 21.01.2005
(51) Int. Cl.: C12N 7/04, A61K 39/12, C12N 15/62, C07K 14/08, C07K 19/00, C12N 15/87, C12N 15/86, C12N 5/10

(54) **EMPTY CAPSIDS (VLPs(-VP4)) OF THE INFECTIOUS BURSAL DISEASE VIRUS (IBDV), OBTAINMENT PROCESS AND APPLICATIONS**
LEERE KAPSIDEN (VLPS(-VP4)) VON DIE INFEKTIÖSE BURSITIS VERUSACHENDEN VIRUS (IBDV), VERFAHREN ZU IHRER HERSTELLUNG UND ANWENDUNGEN
CAPSIDES VIDES (VLPS(-VP4) DU VIRUS DE LA BURSITE INFECTIEUSE(IBDV), SON PROCEDE D'OBTENTION ET SES APPLICATIONS

(30) Priority: 21.01.2004 ES 200400121
(43) Date of publication of application: 04.10.2006
(73) Proprietor: CONSEJO SUPERIOR DE INVESTIGACIONES CIENTIFICAS, 28006 Madrid (ES); Bionostra S.L., 28760 Tres Cantos Madrid (ES)
(72) Inventor: RODRIGUEZ AGUIRRE, José Francisco, Campus de Cantoblanco, 28049 Madrid (ES); RUIZ CASTÓN, José, Campus de Cantoblanco, 28049 Madrid (ES); GONZÁLEZ DE LLANO, María Dolores, Campus de Cantoblanco, 28049 Madrid (ES); OÑA BLANCO, Ana María, Campus de Cantoblanco, 28049 Madrid (ES); ABAITUA ELUSTONDO, Fernando, The Chart, Oxted, Surrey RH8 0TL (GB); LUQUE BUZO, Daniel, Campus de Cantoblanco, 28049 Madrid (ES); RODRIGUEZ FERNANDEZ-ALBA, Juan Ramón, 28760 Tres Cantos-Madrid (ES)
(74) Representative: Gonzalez-Alberto Rodriguez, Natalia
(86) International application number: PCT/EP2005/000694
(87) International publication number: WO 2005/071068

(56) References cited:
- WO-A-01/97839
- US-A- 5 788 970
- HU Y ET AL: "Chimeric infectious bursal disease virus-like particles expressed in insect cells and purified by immobilized metal affinity chromatography" BIOTECHNOLOGY AND BIOENGINEERING. INCLUDING: SYMPOSIUM BIOTECHNOLOGY IN ENERGY PRODUCTION AND CONSERVATION, JOHN WILEY & SONS. NEW YORK, US, vol. 63, no. 6, 20 June 1999 (1999-06-20), pages 721-729, XP002190336 ISSN: 0006-3592
- FERNÁNDEZ-ARIAS A ET AL: "Expression of ORF A1 of infectious bursal disease virus results in the formation of virus-like particles" JOURNAL OF GENERAL VIROLOGY, SOCIETY FOR GENERAL MICROBIOLOGY, READING, GB, vol. 79, no. part 5, May 1998 (1998-05), pages 1047-1054, XP002218365 ISSN: 0022-1317 cited in the application
- MARTINEZ-TORRECUADRADA J L ET AL: "Different Architectures in the Assembly of Infectious Bursal Disease Virus Capsid Proteins Expressed in Insect Cells" VIROLOGY, ACADEMIC PRESS,ORLANDO, US, vol. 278, no. 2, 20 December 2000 (2000-12-20), pages 322-331, XP004435746 ISSN: 0042-6822 cited in the application
- MARTINEZ-TORRECUADRADA J L ET AL: "Structure-dependent efficacy of infectious bursal disease virus (IBDV) recombinant vaccines" VACCINE, BUTTERWORTH SCIENTIFIC. GUILDFORD, GB, vol. 21, no. 23, 4 July 2003 (2003-07-04), pages 3342-3350, XP004429746 ISSN: 0264-410X
- ONA A ET AL: "The C-terminal domain of the pVP2 precursor is essential for the interaction between VP2 and VP3, the capsid polypeptides of infectious bursal disease virus" VIROLOGY, ACADEMIC PRESS,ORLANDO, US, vol. 322, no. 1, 25 April 2004 (2004-04-25), pages 135-142, XP004500338 ISSN: 0042-6822

## Description

### FIELD OF THE INVENTION

The invention is related to empty viral capsids of the infectious bursal disease virus (IBDV), with immunogenic activity against IBDV, constituted by the IBDV VP3 and pVP2 proteins, to the production thereof by means of genetic engineering, and to their applications, particularly in the manufacture of vaccines against the avian disease called infectious bursal disease caused by IBDV, and in the manufacture of gene therapy vectors.

### BACKGROUND OF THE INVENTION

The infectious bursal disease virus (IBDV), also known as Gumboro disease, belongs to the *Birnaviridae* family, infects different bird species and is directly responsible for a severe immunosuppressive disease causing important economic losses in the world poultry industry.

IBDV particles are icosahedral, with T=13 symmetry, they lack an envelope and are formed by a single protein layer. Up until now, the approaches aimed at obtaining an atomic model for IBDV particles have failed. As a result, the structural information available is based on three-dimensional models generated from images obtained by electron cryomicroscopy of the purified virus and of the VLPs. Based on these studies, it has been verified that the outer surface of the particle is formed by a continuous lattice of 260 trimers of the VP2 protein (37 kDa) organized in five different formations. The inner face of the particles contains 200 trimers of the VP3 protein (29 kDa), the latter, independent from one another, are bound to the basal area of the VP2 trimers. It has been suggested that a third polypeptide, VP4 (28 kDa), could also be part of the particles, being located at the base of the pentamers forming the vertices of the icosahedral structure.

The VP2, VP3 and VP4 polypeptides are produced from the proteolytic processing of a polypeptide precursor of a size of 109 kDa. This precursor is auto-catalytically processed, releasing the pVP2 (48 kDa), VP3 and VP4 polypeptides. The VP4 domain, which is located in the central region of the polyprotein, belongs to the Lon protease family and is responsible for the proteolytic cleavage. The pVP2 and VP3 polypeptides are directly responsible for the capsid assembly. The pVP2 product undergoes a last cleavage at its C-terminal end before giving rise to the mature form of the protein, VP2, which is the one found in purified particles (Da Costa, B., Chevalier, C., Henry, C., Huet, J. C., Petit, S., Lepault, J., Boot, H. & Delmas, B. (2002). The capsid of infectious bursal disease virus contains several small peptides arising from the maturation process of pVP2. Journal of Virology 76:2393-2402). This pVP2 processing is necessary for the correct formation of the capsids and requires the presence of VP3, although the responsible protease has not yet been identified (Maraver, A., Oña, A., Abaitua, F., González, D., Clemente, R., Diaz-Ruiz, A., Caston, J. R., Pazos, F. & Rodríguez, J. F. (2003). The oligomerization domain of VP3, the scaffolding protein of infectious bursal disease virus, plays a critical role for capsid formation. Journal of Virology 77:6438-49).

Conventional vaccines used for the control of infectious bursal disease are based on the use of strains, with different degrees of virulence, of IBDV itself grown in cell culture or in embryonated eggs. The extracts containing the infectious material are subjected to chemical inactivation processes to produce inactivated vaccines, or else are used directly to produce live attenuated vaccines (Sharma, J. M., Kim, I. J., Rautenschlein, S. & Yeh, H. Y. (2000). Infectious bursal disease virus of chickens: pathogenesis and immunosuppression. Developmental and Comparative Immunology 24:223-235; van den Berg TP, Eterradossi N, Toquin D, Meulemans G. 2000. Rev Sci Tech 2000, 19:509-543). This latter type of vaccines has the typical drawbacks associated with the use of live attenuated vaccines, specifically, the risk of mutations reverting the virulence of the virus or making it lose its immunogenicity.

Recombinant subunit vaccines containing the IBDV protein VP2 expressed in several expression systems, for example, bacteria, yeasts or baculovirus, usually in fusion protein form, have been disclosed. The results obtained in chicken immunization tests with said vaccines have not been completely satisfactory.

Empty viral capsids or virus-like particles (VLPs) constitute an alternative to the use of live attenuated vaccines and of recombinant subunit vaccines. VLPs are obtained by self-assembly of the subunits constituting the viral capsid and mimicking the structure and antigenic properties of the native virion, even though they lack genetic material, as a result of which they are incapable of replicating themselves. Apart from their application for vaccination purposes, VLPs can be used as vectors of molecules of biological interest, for example, nucleic acids, peptides or proteins. By way of illustration, parvovirus VLPs (US 6,458,362) or human immunodeficiency syndrome (HIV) VLPs (US 6,602,705), can be mentioned.

Morphogenesis is a vital process for the viral cycle requiring successive steps associated to modifications in the polypeptide precursors. As a result, viruses have developed strategies allowing the sequential and correct interaction between each one of their components. One of these strategies, frequently used by icosahedral viruses, is the use of polypeptides coming from a single polyprotein as the base of their structural components. In these cases, the suitable proteolytic processing of said polyprotein plays a crucial role in the assembly process.

This concept for the assembly of IBDV capsids has been demonstrated in earlier work (Fernández-Arias, A., Risco, C., Martínez, S., Albar, J. P. & Rodríguez, J. F. (1998). Expression of ORF A1 of infectious bursal disease virus results in the formation of virus-like particles. Journal of General Virology 79, 1047-1054). Expression of the gene encoding for the IBDV polyprotein in eukaryotic cells gives rise to the formation of VLPs that are completely morphologically and biochemically indistinguishable from the IBDV virions. It has also been shown that the assembly of the capsids requires only the synthesis and correct processing of the viral polyprotein and is independent of the presence of the viral genome or of other proteins encoded by the viral genome, such as VP5 and VP1.

Parallelly to the formation of capsids, the IBDV VP4 product is able to self-assemble in tubular structures of 20 nm in diameter. These tubules, known as type II tubules, are partially copurified with the viral particles. Other experiments have demonstrated that obtaining IBDV VLPs, using recombinant baculoviruses (rBVs) for polyprotein expression, is extremely inefficient, the accumulation of large amounts of type I tubules in the cytosol of the infected cells being obtained (Martínez-Torrecuadrada, J. L., Castón, J. R., Castro, M., Carrascosa, J. L., Rodríguez, J. F. & Casal, J. I. (2000). Different architectures in the assembly of infectious bursal disease virus capsid proteins expressed in insect cells. Virology 278:322-331; Chevalier, C., Lepault, J., Erk, I., Da Costa, B. & Delmas, B. (2002). The maturation process of pVP2 requires assembly of infectious bursal disease virus capsids. Journal of Virology 76:2384-2392). It has recently been demonstrated that this is due to the proteolytic cleavage to which the VP3 protein is subjected when it is synthesized in insect cells. Said proteolysis is almost completely prevented by the formation of VP3/VP1 complexes (Maraver, A., Oña, A., Abaitua, F., González, D., Clemente, R., Diaz-Ruiz, A., Caston, J. R., Pazos, F. & Rodríguez, J. F. (2003). The oligomerization domain of VP3, the scaffolding protein of infectious bursal disease virus, plays a critical role for capsid formation. Journal of Virology 77:6438-6449).

Therefore, the results obtained to date from the IBDV gene expression in different recombinant systems has allowed concluding that: (i) the assembly process is independent of the presence of genetic material of the virus, (ii) only the polypeptides encoded by the polyprotein gene are necessary for the assembly, and (iii) the assembly requires a coordinated interaction between the pVP2 and VP3 peptides.

However, it is not known if the VP2/VP3 interaction is established between VP2 and VP3 domains of the polyprotein precursor when it has not yet undergone modifications, or on the contrary, if this interaction occurs after the processing of the precursor. Furthermore, current information does not exclude the possibility that VP4 could play a relevant role in the morphogenesis of the viral capsid. In fact, IBDV VLPs formed by assembly of the IBDV VP2, VP3 and VP4 proteins have been disclosed (US 6,528,063, US 5,788,970 and JP 5194597).

On the other hand, information regarding IBDV protein expression by means of genetic engineering in different cell models is scarce. IBDV protein expression in insect cells, bacteria and yeasts has been disclosed. Jagadish et al. (Jagadish MN, Vaughan PR, Irving RA, Azad AA, Macreadie IG. (1990). Expression and characterization of infectious bursal disease virus polyprotein in yeast. Gene 9:179-186; Macreadie IG, Vaughan PR, Chapman AJ, McKern NM, Jagadish MN, Heine HG, Ward CW, Fahey KJ, Azad AA. (1990). Passive protection against infectious bursal disease virus by viral VP2 expressed in yeast. Vaccine 8:549-552) disclose IBDV VP2 expression in yeasts. The disclosed results indicate that the viral polyprotein expression in two yeast species, *Saccharomyces cerevisiae* and *Saccharomyces pombe,* is very inefficient, a large variety of protein products of different molecular mass being accumulated. The failure to obtain protein products of the expected size and the inability to detect structures produced due to the assembly of the latter was attributed to two possible causes: (i) possible toxicity of the IBDV protease (VP4) in this system; and/or (ii) the inefficiency of the expression system to carry out a correct transcription and/or translation of the IBDV polyprotein. Recently, Pitcovski et al. (Pitcovski J., Gutter B, Gallili G, Goldway M, Perelman B, Gross G, Krispel S, Barbakov M, Michael A. (2003). Vaccine 21:4736-4743) have disclosed the IBDV VP2 expression in *Pichia pastoris* and the immunization of chickens with a material comprising the recombinant protein (rVP2) in partially purified form. In no case has the obtainment of IBDV VLPs in yeast been disclosed.

Earlier work developed by the inventors has enabled establishing systems for obtaining IBDV VLPs using different eukaryotic expression vectors. These vectors have been used for IBDV polyprotein expression in the absence or presence of the viral VP1 RNA polymerase. The biochemical characterization of purified VLPs demonstrates that they contain pVP2, VP2 and VP3 proteins when only the viral polyprotein is expressed, and the pVP2, VP2, VP3 and VP1 proteins when the simultaneous expression of the polyprotein and viral RNA polymerase is carried out (Fernández-Arias, A., Risco, C., Martínez, S., Albar, J. P. & Rodríguez, J. F. (1998). Expression of ORF A1 of infectious bursal disease virus results in the formation of virus-like particles. Journal of General Virology 79: 1047-1054; Martínez-Torrecuadrada, J. L., Castón, J. R., Castro, M., Carrascosa, J. L., Rodríguez, J. F. & Casal, J. I. (2000). Different architectures in the assembly of infectious bursal disease virus capsid proteins expressed in insect cells. Virology 278: 322-331; Maraver, A., *et al.,* (2003) cited *supra;* Lombardo, E., Maraver, A., Castón, J. R., Rivera, J., Fernández-Arias, A., Serrano, A., Carrascosa, J. L. & Rodríguez, J. F. (1999). VP1, the putative RNA-dependent RNA polymerase of infectious bursal disease virus, forms complexes with the capsid protein VP3, leading to efficient encapsidation into virus-like particles. Journal of Virology 73: 6973-6983). However, VLPs solely based on IBDV pVP2 and VP3, or their potential use for vaccine purposes or as vehicles of biological products of interest, have not been previously disclosed.

### SUMMARY OF THE INVENTION

The invention is faced with the problem of providing new effective and safe vaccines against the infectious bursal disease virus (IBDV).

The solution provided by this invention is based on it being possible to obtain correctly assembled IBDV VLPs by means of the simultaneous expression of the IBDV pVP2 and VP3 polypeptides as independent genes and as the only representation of IBDV proteins in a gene expression system. Said VLPs are formed by self-assembly of only IBDV pVP2 and VP3, whereby they lack IBDV VP4 and, for this reason, are called VLP(-VP4) (singular) or VLPs(-VP4) (plural) in this description. Said VLPs(-VP4) can be used for example for therapeutic or diagnostic purposes, for example, in the manufacture of vaccines to protect birds from the infection caused by IBDV, or in the manufacture of gene therapy vectors.

The obtained results allow conceiving two new conclusions to the understanding of the IBDV assembly pattern: (i) the interactions between the pVP2/VP3 polypeptides result in an efficient assembly of the IBDV particles without needing expression of the whole polyprotein, and (ii) the VP4 polyprotein is not required for capsid formation.

These results have allowed designing a new strategy or process for the efficient production of IBDV VLPs containing antigenically relevant protein elements so as to induce an immune response. This strategy is based on the use of a gene expression system or vector allowing the coexpression of pVP2 and VP3 polypeptides as independent genes and preventing the synthesis of the polyprotein precursor of said polypeptides as well as the presence of the VP4 polypeptide during the empty viral capsid assembly process.

The expression and obtainment of IBDV VLPs, particularly VLPs(-VP4), in insect cells is described in a particular embodiment, while in another particular embodiment, said VLPs(-VP4) are obtained in yeasts, with a very high yield and a very low economic cost.

The vaccines obtained using said VLPs(-VP4) have a number of advantages since, on one hand, the handling of highly infectious material is prevented, the potential risk of the occurrence of new IBDV mutants is prevented and the use of a live virus in poultry farms is eliminated, thus preventing the risk of spreading IBDV vaccine strains to the environment, and on the other hand, it enables the development of differential diagnostic systems to discriminate between vaccinated and infected animals. These diagnostic systems are based on the detection of antibodies against VP2 and VP4 proteins. Animals with IBDV develop a strong humoral response to both proteins. However animals immunized with VLPs(-VP4) only have antibodies against the VP2 protein.

Consequently, an object of the present invention consists of an empty IBDV viral capsid, VLP(-VP4), with immunogenic activity against infection in IBDV, characterized in that it is constituted by self-assembly of only IBDV pVP2 and VP3 proteins.

A further aspect of this invention is related to a process for producing said IBDV VLPs(-VP4) provided by this invention, based on the gene coexpression of said IBDV pVP2 and VP3 proteins as two independent genes.

The nucleic acids, gene constructs, expression systems and host cells developed for implementing said process of producing said IBDV VLPs(-VP4), as well as their use for the production of said IBDV VLPs(-VP4), constitute further aspects of the present invention.

Said IBDV VLPs(-VP4) have the ability to immunize animals, particularly birds, against the avian disease caused by IBDV, as well as the ability to incorporate in vectors or vehicles molecules of biological interest, for example, polypeptides, proteins, nucleic acids, etc. In a particular embodiment, said IBDV VLPs(-VP4) can be used in the manufacture of a vaccine to protect birds against the virus causing the avian disease known as infectious bursal disease (IBDV). Virtually any bird, preferably those avian species of economic interest, for example, chickens, turkeys, geese, ganders, pheasants, quails, ostriches, etc., can be immunized against the infection caused by IBDV with the vaccines provided by this invention. In another particular embodiment, said IBDV VLPs(-VP4) can internally incorporate into vehicles products with biological activity, for example, nucleic acids, peptides, proteins, drugs, etc., whereby they can be used in the manufacture of gene therapy vectors.

Therefore, in a further aspect, the present invention is related to the use of said IBDV VLPs(-VP4), in the manufacture of medicaments, such as vaccines and gene therapy vectors. Said vaccines and vectors constitute further aspects of the present invention. In a particular embodiment, said vaccine is a vaccine useful for protecting birds from the infection caused by IBDV. In a specific embodiment, said birds are selected from the group formed by chickens, turkeys, geese, ganders, pheasants, quails and ostriches, preferably chickens.

### BRIEF DESCRIPTION OF THE FIGURES

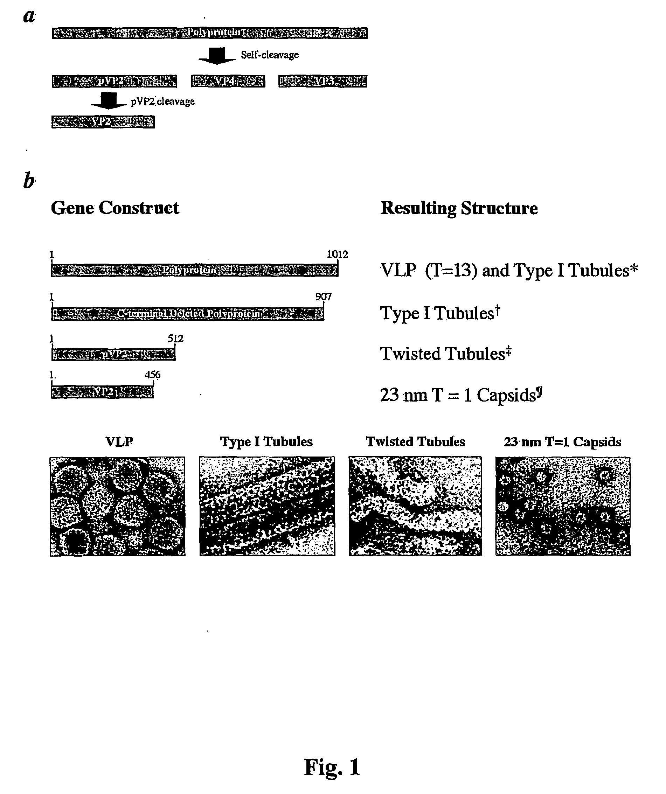

**Figure 1.** (*a*) The diagram schematizes the proteolytic processing steps necessary for the formation of mature VP2 and VP3 capsid proteins from the polyprotein precursor. (b) The diagram reflects the different genetic constructs derived from the IBDV polyprotein described up until now, as well as the structures produced by means of its expression in different heterologous systems. The numbers indicate the position corresponding to the first and last amino acid residue of the polyprotein present in each one of the constructs. The lower portion of the figure shows images obtained by means of transmission electron microscopy of the structures obtained by means of expression of the different constructs. The bar corresponds to 50 nm. The data has been taken from the following literature references: Fernández-Arias *et al.,* (1998), cited *supra;* Maraver *et al.,* (2003), cited *supra;* Martínez-Torrecuadrada *et al.,* (2000), cited *supra;* Castón et al., 2001. C terminus of infectious bursal disease virus major capsid protein VP2 is involved in definition of the t number for capsid assembly. Journal of Virology 75, 10815-10828.

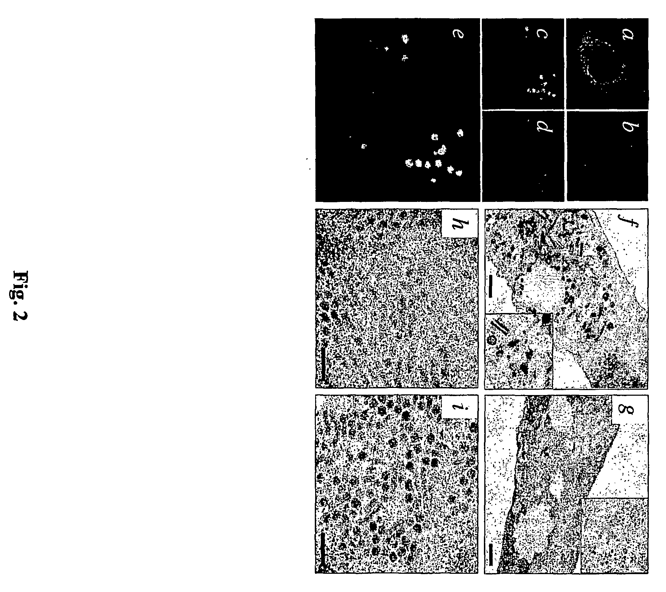

**Figure 2. Microscopic analysis of H5 insect cells coexpressing pVP2 and VP3.** The pVP2 and VP3 protein subcellular distribution was analyzed by means of confocal immunomicroscopy. Cells infected with the FB/pVP2 (a), FB/VP3 (b), or FBD/pVP2-VP3 (c-e) rBVs were incubated with anti-pVP2 rabbit serum and anti-VP3 rat serum. Then the cells were incubated with goat anti-rabbit IgG serum coupled to Alexa 488 (red) and goat anti-rat IgG serum coupled to Alexa 594 (green). The cores were stained with To-Pro 3 (blue). (e) Overlaying of the images shown in panels (c) and (d). Electron microscopy images corresponding to sections of H5 cells infected with different genetic constructs derived from the IBDV polyprotein. (f) Low-magnification image of an H5 cell infected with a parental Fb virus. The insert corresponds to an enlarged detail of the area indicated by the box. (g) Low-magnification image of an H5 cell infected with the FBD/pVP2-VP3 virus. The insert corresponds to an enlarged detail of the area indicated by the box. (h) High-magnification image of an H5 cell infected with the FBD/pVP2-VP3 virus showing the formation of IBDV structures in detail. (i) High-magnification image of a BSC1 cell infected with the VTLacOI/POLY recombinant vaccine virus showing structures similar to those detected in panel (h). The bars indicate 600 nm (panels f and g) and 200 nm (panels h and i).

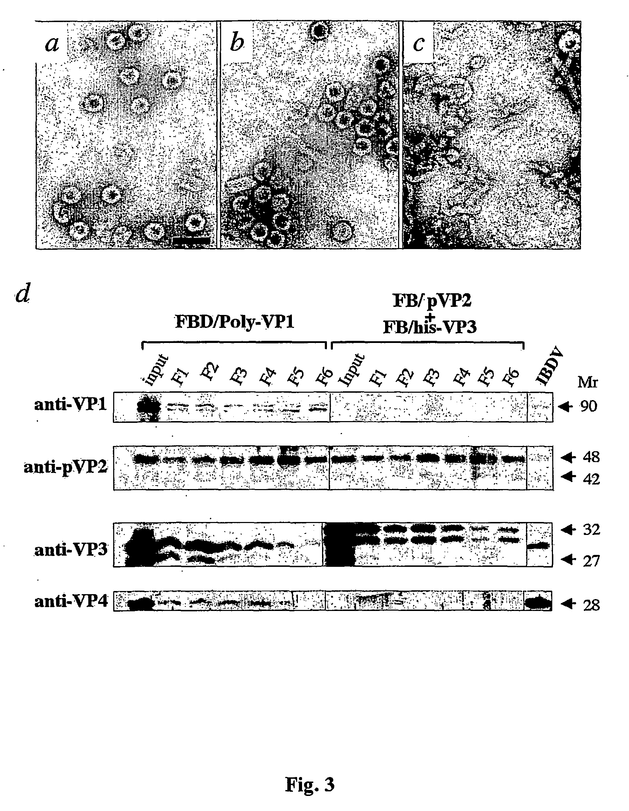

**Figure 3. Structural and biochemical characterization of the structures derived from IBDV produced in insect cells coinfected with the (rBV) FB/pVP2 + FB/his-VP3 recombinant baculoviruses.** Cells coinfected with FB/pVP2 and FB/his-VP3 rBVs, or infected with the FBD/Poly-VP1 or FB/pVP2 virus, were used to purify structures derived from IBDV by means of centrifugation on sucrose gradients. Panels (*a*), (*b*), and (*c*) show transmission electron microscopy images corresponding to fraction 4 of the gradients obtained from infections with FBD/Poly-VP1, FB/pVP2+FB/his-VP3, and FB/pVP2, respectively. Panel (d) shows the results of a Western blot analysis of the sucrose gradients corresponding to the cultures infected with FBD/Poly-VP1 and FB/pVP2+FB/his-VP3, respectively. The total extracts (input) and the different fractions of the sucrose gradients (fraction F1 corresponds to the bottom of the gradient) were analyzed by means of Western blot using specific sera against the IBDV VP1, pVP2, VP3, and VP4 proteins, respectively. The molecular mass of the immunoreactive polypeptides is indicated in kDa.

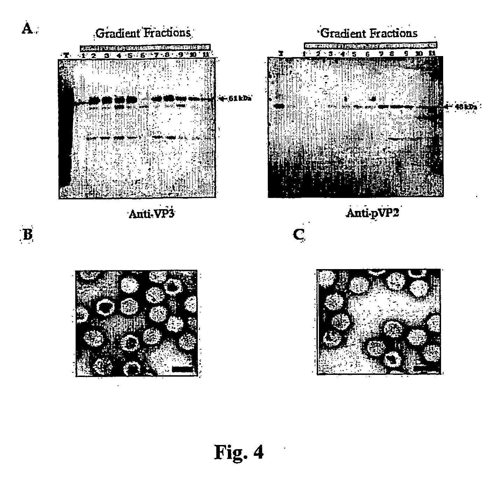

**Figure 4. Biochemical and structural characterization of IBDV VLPs produced in *S. cerevisiae* transformed with the plasmid pESCURA/pVP2-VP3-GFP.** A *S. cerevisiae* culture transformed with the plasmid pESCURA/pVP2-VP3-GFP was grown at 30°C in a medium supplemented with the inducer galactose. At 18 hours, the culture was harvested and centrifuged. The resulting sediment was processed by means of fractioning in a 25-50% linear sucrose gradient. A) Biochemical analysis of samples corresponding to the sediment before fractioning (T) as well as the different fractions of the sucrose gradient. The samples were analyzed by means of SDS-PAGE and Western blot using specific antibodies against VP3 (anti-VP3) and pVP2 (anti-pVP2) proteins. The arrows indicate the positions of the immunoreactive bands corresponding to the VP3-GFP (61 kDa) and pVP2 (48 kDa) proteins, respectively. B) The structural analysis of the obtained samples was carried out by means of TEM. The image corresponds to a micrography obtained from an aliquot corresponding to the mixture of fractions 7, 8 and 9 of the sucrose gradient. The sample was stained with uranyl acetate and observed by means of TEM. The bar corresponds to 65 nm. C) VLPs sample obtained by means of the IBDV polyprotein expression in mammal cells by means of infection with the VT7/Poly recombinant vaccine virus (Fernández-Arias *et al.,* (1998), cited *supra*). The bar corresponds to 65 nm.

### DETAILED DESCRIPTION OF THE INVENTION

In a first aspect, the invention provides an empty capsid of the infectious bursal disease virus (IBDV), hereinafter VLP(-VP4) of the invention, characterized in that it is constituted by assembly of only IBDV pVP2 proteins and IBDV VP3 proteins.

The term "IBDV", as it is used in the present invention, refers to the different IBDV strains belonging to any of the known serotypes (1 or 2) [by way of illustration, see the review carried out by van den Berg TP, Eterradossi N, Toquin D, Meulemans G., en Rev Sci Tech 2000 19: 509-43] and the terms "IBDV pVP2 protein" and "IBDV VP3 protein" refer to the different forms of the pVP2 and VP3 proteins representative of any of the mentioned IBDV strains [NCBI protein databank], according to the definition made by Sánchez and Rodríguez (1999) (Sánchez AB, Rodríguez JF. Proteolytic processing in infectious bursal disease virus: identification of the polyprotein cleavage sites by site-directed mutagenesis. Virology. 1999 Sep 15; 262(1):190-199), as well as proteins substantially homologous to said IBDV pVP2 and VP3 proteins, i.e. proteins the amino acid sequences of which have a degree of identity regarding said IBDV pVP2 and VP3 proteins of at least 60%, preferably of at least 80%, more preferably of at least 90% and even more preferably of at least 95%.

The IBDV pVP2 protein present in the VLP(-VP4) of the invention can be any pVP2 protein representative of any IBDV strain, for example, the full-length pVP2 protein of IBDV Soroa strain [NCBI, access number AAD30136]

The IBDV VP3 protein present in the VLP(-VP4) of the invention can be any VP3 protein representative of any IBDV strain, for example, the full-length VP3 protein of IBDV Soroa strain [NCBI, access number AAD30136].

In a particular embodiment, the VLPs(-VP4) of the invention have a diameter of 65-70 nm and a polygonal contour indistinguishable from the IBDV VLPs obtained in other expression systems (Figure 4C).

The VLPs(-VP4) of the invention can be obtained by means of the simultaneous expression of said IBDV pVP2 and VP3 proteins in suitable host cells. Said suitable host cells are cells containing the nucleotide sequence encoding the IBDV pVP2 protein and the nucleotide sequence encoding the IBDV VP3 protein, either in a single gene construct or in two gene constructs. In a particular embodiment, said suitable host cells are cells that are transformed, transfected or infected with a suitable expression system, such as an expression system comprising a gene construct, wherein said gene construct comprises the nucleotide sequence encoding for the IBDV pVP2 protein and the nucleotide sequence encoding the IBDV VP3 protein, or else alternatively with an expression system comprising a first gene construct comprising the nucleotide sequence encoding for the IBDV pVP2 protein, and a second gene construct comprising the nucleotide sequence encoding for the IBDV VP3 protein.

Therefore, in another aspect, the invention provides a nucleic acid, the nucleotide sequence of which comprises the nucleotide sequence encoding for said IBDV pVP2 protein and the nucleotide sequence encoding for said IBDV VP3 protein in the form of two independent genes. More specifically, the nucleic acid provided by this invention is characterized in that its nucleotide sequence is constituted by (i) a nucleotide sequence comprising the open reading frame corresponding to the IBDV pVP2 protein and (ii) a nucleotide sequence comprising the open reading frame corresponding to the IBDV VP3 protein. One feature of the nucleic acid provided by this invention is that it lacks the nucleotide sequence comprising the open reading frame corresponding to the IBDV VP4 protein. As it is used in this description, the term "open reading frame corresponding to the pVP2 protein" or "open reading frame corresponding to the IBDV VP3 proteins" includes, apart from the nucleotide sequences of said open reading frames, other open reading frames analogous to the same encoding frames of the IBDV pVP2 and VP3 proteins. As it is used herein, the term "analogous" intends to include any nucleotide sequence which can be isolated or constructed on the base of the encoding nucleotide sequence of IBDV pVP2 and VP3, for example by means of the introduction of conservative or non-conservative nucleotide replacements, including the insertion of one or more nucleotides, the addition of one or more nucleotides at any of the ends of the molecule, or the deletion of one or more nucleotides at any end or inside of the sequence. Generally, a nucleotide sequence analogous to another nucleotide sequence is substantially homologous to said nucleotide sequence. In the sense used in this description, the expression "substantially homologous" means that at the nucleotide level the nucleotide sequences in question have a degree of identity of at least 60%, preferably of at least 80%, more preferably of at least 90%, and even more preferably of at least 95%.

In another aspect, the invention provides a gene construct comprising a nucleic acid provided by this invention, i.e. a nucleic acid the nucleotide sequence of which is constituted by (i) a nucleotide sequence comprising the open reading frame corresponding to the IBDV pVP2 protein and (ii) a nucleotide sequence comprising the open reading frame corresponding to the IBDV VP3 protein. Said gene construct lacks the nucleotide sequence comprising the open reading frame corresponding to the IBDV VP4 protein.

In another aspect, the invention provides an expression vector or system selected from:
a) an expression system comprising a gene construct provided by this invention, operatively bound to transcription, and optionally translation, control elements, wherein said gene construct comprises the nucleotide sequence comprising the open reading frame corresponding to the IBDV pVP2 protein and the nucleotide sequence comprising the open reading frame corresponding to the IBDV VP3 protein; and
b) an expression system comprising two gene constructs, a first gene construct comprising the open reading frame corresponding to the IBDV pVP2 protein, operatively bound to transcription, and optionally translation, control elements, and a second gene construct comprising the open reading frame corresponding to the IBDV VP3 protein, operatively bound to transcription, and optionally translation, control elements.

In a particular embodiment, the expression system provided by this invention comprises a gene construct comprising (i) a nucleotide sequence comprising the open reading frame or encoding region corresponding to the IBDV pVP2 protein and (ii) a nucleotide sequence comprising the open reading frame or encoding region corresponding to the IBDV VP3 protein, wherein said gene construct is operatively bound to transcription, and optionally translation, control elements.

In another particular embodiment, the expression system provided by this invention comprises (i) a first gene construct, operatively bound to transcription, and optionally translation, control elements, said first gene construct comprising a nucleotide sequence comprising the open reading frame or encoding region corresponding to the IBDV pVP2 protein, and (ii) a second gene construct, operatively bound to transcription, and optionally translation, control elements, said second gene construct comprising a nucleotide sequence comprising the open reading frame or encoding region corresponding to the IBDV VP3 protein.

The transcription, and optionally translation, control elements present in the expression system provided by this invention include promoters, directing the transcription of the nucleotide sequences of interest to which they are operatively linked, and other sequences necessary or suitable for the transcription and its suitable regulation in time and place, for example, start and end signals, cleavage sites, polyadenylation signal, replication origin, transcriptional activators (enhancers), transcriptional silencers (silencers), etc.

Virtually any suitable expression system or vector can be used in the generation of the expression system provided by this invention. By way of illustration, said suitable expression or vector systems can be selected, according to the conditions and needs of each specific case, from plasmids, bacmids, yeast artificial chromosomes (YACs), bacteria artificial chromosomes (BACs), bacteriophage P1-based artificial chromosomes (PACs), cosmids, or viruses, which can further have a heterologous replication origin, for example, bacterial or of yeast, so that it may be amplified in bacteria or yeasts, as well as a marker usable for selecting the transfected cells different from the gene or genes of interest. These expression systems or vectors can be obtained by conventional methods known by persons skilled in the art [Sambrook, J., Fritsch, E.F., and Maniatis, T. (1989). Molecular cloning: a laboratory manual, 2nd ed. Cold Spring Harbor Laboratory] and form part of the present invention. In a particular embodiment, said expression or vector system is a plasmid, such as a plasmid suitable for transforming yeasts, for example, the plasmid called pESCURA/pVP2-VP3-GFP (Example 2), or a virus, such as a recombinant baculovirus (rBV), for example, the rBV called FBD/pVP2-his-VP3 (Example 1.2), simultaneously expressing both proteins (IBDV pVP2 and his-VP3) in insect cells during the replication cycle, or the rBVs called FB/pVP2 and FB/his-VP3 (Example 1.1) expressing the IBDV pVP2 and his-VP3 proteins, respectively, when coinfecting insect cells, IBDV VLPs(-VP4) being obtained.

In another aspect, the invention provides a host cell containing the encoding nucleotide sequence of the IBDV pVP2 protein and the encoding nucleotide sequence of the IBDV VP3 protein, either in a single gene construct or in two different gene constructs. In a particular embodiment, said host cell is a host cell that is transformed, transfected or infected with (i) an expression system provided by this invention comprising either a gene construct wherein said gene construct comprises the nucleotide sequence encoding for said IBDV pVP2 protein and the nucleotide sequence encoding for said IBDV VP3 protein, or else alternatively with (ii) an expression system comprising a gene construct comprising the nucleotide sequence encoding for said IBDV pVP2 protein and another gene construct comprising the nucleotide sequence encoding for said IBDV VP3 protein.

In a particular embodiment, the host cell provided by this invention is a host cell that is transformed, transfected or infected with an expression system comprising a gene construct comprising (i) a nucleotide sequence comprising the open reading frame or encoding region corresponding to the IBDV pVP2 protein and (ii) a nucleotide sequence comprising the open reading frame or encoding region corresponding to the IBDV VP3 protein, wherein said gene construct is operatively bound to transcription, and optionally translation, control elements.

In another particular embodiment, the host cell provided by this invention is a host cell that is transformed, transfected or infected with a first gene construct, operatively bound to transcription, and optionally translation, control elements, said first gene construct comprising a nucleotide sequence comprising the open reading frame or encoding region corresponding to the IBDV pVP2 protein, and with a second gene construct, operatively bound to transcription, and optionally translation, control elements, said second gene construct comprising a nucleotide sequence comprising the open reading frame or encoding region corresponding to the IBDV VP3 protein.

Virtually any host cell susceptible to being transformed, transfected or infected by an expression system provided by this invention can be used, for example, mammal cells, bird cells, insect cells, yeasts, etc; however, in a particular embodiment, said host cell is selected from yeasts and insect cells. Yeasts are suitable due to the simplicity and production cost. Insect cells are suitable when the expression system comprises one or two recombinant baculoviruses (rBV). The use of rBV is advantageous due to biosafety issues related to the host range of the baculoviruses, incapable of replicating in other cell types which are not insect cells.

In a particular embodiment, the invention provides a host cell, such as a yeast, for example, *Saccharomyces cerevisiae, Saccharomyces pombe,* etc., transformed with an expression system, such as a plasmid or an expression vector, comprising a gene construct provided by this invention comprising the nucleotide sequence encoding for said IBDV pVP2 protein and the nucleotide sequence encoding for the IBDV VP3 protein.

In another particular embodiment, the invention provides a host cell, such as an insect cell, infected with an expression system, such as a recombinant baculovirus, comprising a gene construct provided by this invention comprising the nucleotide sequence encoding for said IBDV pVP2 protein and the nucleotide sequence encoding for the IBDV VP3 protein.

In another particular embodiment, the invention provides a host cell, such as an insect cell, coinfected with an expression system comprising a first recombinant baculovirus comprising a gene construct provided by this invention comprising the nucleotide sequence encoding for said IBDV pVP2 protein and with a second recombinant baculovirus comprising a gene construct provided by this invention comprising the nucleotide sequence encoding for the IBDV VP3 protein.

In another aspect, the invention provides a process for the production of VLPs(-VP4) of the invention comprising culturing a host cell provided by this invention containing the encoding nucleotide sequence of said IBDV pVP2 protein and the encoding nucleotide sequence of said IBDV VP3 protein, either in a single gene construct or in two different gene constructs, and simultaneously expressing said IBDV pVP2 and VP3 proteins, and if so desired, recovering said VLPs(-VP4) of the invention. In a particular embodiment, said host cell provided by this invention is a cell that is transformed, transfected or infected with a suitable expression system, such as an expression system comprising a gene construct, wherein said gene construct comprises the nucleotide sequence encoding for said IBDV pVP2 protein and the nucleotide sequence encoding for said IBDV VP3 protein, or else alternatively with an expression system comprising a first gene construct comprising the nucleotide sequence encoding for said IBDV pVP2 protein and a second gene construct comprising the nucleotide sequence encoding for said IBDV VP3 protein.

Said process therefore comprises the gene coexpression of said IBDV pVP2 and VP3 proteins as two independent genes. After the simultaneous expression of said proteins (IBDV pVP2 and VP3) in said cells, the expressed proteins are assembled and form the VLPs(-VP4) of the invention, which can be isolated or withdrawn from the medium and purified if desired. The isolation and purification of said VLPs(-VP4) of the invention can be carried out by means of conventional methods, for example, by means of fractioning on sucrose gradients.

In a particular embodiment, the simultaneous gene coexpression of IBDV pVP2 and VP3 proteins is carried out by means of the use of an rBV allowing the simultaneous expression of said proteins from two independent chimeric genes in insect cells. In this case, the production of VLPs(-VP4) of the invention can be carried out by means of a process comprising, first, the obtainment of a gene expression system made up of an rBV containing a gene construct simultaneously encoding for said IBDV pVP2 and VP3 proteins, such as the rBV called FBD/pVP2-his-VP3 (Example 1.2), or alternatively the obtainment of an rBV containing a gene construct encoding for the IBDV pVP2 protein and the obtainment of another rBV containing a gene construct encoding for said IBDV VP3 protein, such as the rBVs called FB/pVP2 and FB/his-VP3 (Example 1.1), followed by the infection of insect cells with said expression system based on said recombinant baculovirus(es), expression of the recombinant proteins and if so desired, isolation of the VLPs(-VP4) of the invention formed by assembly of said IBDV pVP2 and VP3 proteins, and optionally subsequent purification of said VLPs(-VP4) of the invention.

The construction of a recombinant baculovirus allowing the independent expression of the IBDV pVP2 and VP3 proteins can be carried out by any person skilled in the art based on that described herein and on the state of the art concerning this technology (Cold Spring Harbor, N.Y.; Leusch MS, Lee SC, Olins PO. 1995. A novel host-vector system for direct selection of recombinant baculoviruses (bacmids) in Escherichia coli. Gene 160: 191-4; Luckow VA, Lee SC, Barry GF, Olins PO. 1993. Efficient generation of infectious recombinant baculoviruses by site-specific transposon-mediated insertion of foreign genes into a baculovirus genome propagated in Escherichia coli. J Virol 67: 4566-79).

In another particular embodiment, the gene coexpression of the IBDV pVP2 and VP3 proteins is carried out by means of a vector allowing the expression of said proteins in yeast cells. In this case, the production of VLPs(-VP4) of the invention can be carried out by means of a process comprising, first, the obtainment of a gene expression system made up of a plasmid containing a gene construct simultaneously encoding for the IBDV pVP2 and VP3 proteins, followed by the transformation of yeasts with said expression system, expression of the recombinant proteins, and if so desired, isolation of the VLPs(-VP4) of the invention formed by assembly of said IBDV pVP2 and VP3 proteins, and optionally subsequent purification of said VLPs(-VP4) of the invention. In a specific embodiment, the suitable expression system for transforming yeasts is based on a pESC Yeast (Stratagene) expression system such as, for example, the plasmid pESCURA/pVP2/VP3-GFP (Example 2) containing a gene construct encoding for the IBDV pVP2 and VP3-GFP proteins.

The obtainment of yeasts transformed with a gene construct or with a suitable expression system or vector allowing the simultaneous expression of the IBDV pVP2 and VP3 proteins can be carried out by any person skilled in the art based on that described herein and on the state of the art concerning this technology (pESC epitope tagging vectors Instructions manual. Stratagene www.stratagene.com; Sambrook, J., Fritsch, E.F., and Maniatis, T. (1989). Molecular cloning: a laboratory manual, 2nd ed. Cold Spring Harbor Laboratory).

In another aspect, the invention is related to the use of the gene expression system provided by this invention for producing and obtaining the VLPs(-VP4) of the invention.

The VLPs(-VP4) of the invention can be used to immunize animals, particularly birds, *per se* or as vectors or vehicles of molecules with biological activity, for example, polypeptides, proteins, nucleic acids, drugs, etc., whereby they can be used for therapeutic or diagnostic purposes. In a particular embodiment, said molecules with biological activity include antigens or immune response inducers in animals or humans to whom they are supplied, or drugs which can be released in their specific action site, or nucleic acid sequences, all being useful in gene therapy and intended for being introduced inside the suitable cells.

Therefore, in another aspect, the invention is related to the use of the VLPs(-VP4) of the invention in the manufacture of medicaments such as vaccines, gene therapy vectors (delivery systems), etc. In a particular embodiment, said medicament is a vaccine intended for conferring protection to animals, particularly birds, against the infectious bursal disease virus (IBDV). In another particular embodiment, said medicament is a gene therapy vector.

In another aspect, the invention provides a vaccine comprising a therapeutically effective amount of VLPs(-VP4) of the invention, optionally together with one or more pharmaceutically acceptable adjuvants and/or vehicles. Said vaccine is useful for protecting animals, particularly birds, against the infectious bursal disease virus (IBDV). In a particular embodiment, said birds are selected from the group formed by chickens, turkeys, geese, ganders, pheasants, quails and ostriches. In a preferred embodiment, the vaccine provided by this invention is a vaccine useful for protecting chickens from the infection caused by IBDV.

In the sense used in this description, the expression "therapeutically effective amount" refers to the amount of VLPs(-VP4) of the invention calculated for producing the desired effect and will generally be determined, among others, by the characteristics of the VLPs(-VP4) and the immunization effect to be achieved.

The pharmaceutically acceptable adjuvants and vehicles which can be used in said vaccines are those adjuvants and vehicles known by the persons skilled in the art and normally used in the manufacture of vaccines.

In a particular embodiment, said vaccine is prepared in form of an aqueous solution or suspension in a pharmaceutically acceptable diluent, such as saline solution, phosphate-buffered saline solution (PBS), or any other pharmaceutically acceptable diluent.

The vaccine provided by this invention can be administered by any suitable administration route which results in a protective immune response against the heterologous sequence or epitope used, to which end said vaccine will be formulated in the dosage form suited to the chosen administration route. In a particular embodiment, the administration of the vaccine provided by this invention is carried out parenterally, for example, intraperitoneally, subcutaneously, etc.

The following Examples illustrate the invention and should not be considered limiting of the scope thereof.

### EXAMPLE 1

### Obtaining VLPs(-VP4) by means of the coexpression of pVP2 (pVPX) and VP3 in insect cells

### 1.1 Obtaining VLPs(-VP4) by means of the coexpression of pVP2 (pVPX) and VP3 with two independent rBVs in insect cells

The results of a series of experiments designed to analyze the possibility of obtaining IBDV VLPs from a strategy avoiding the synthesis of the IBDV polyprotein and, therefore, the presence of the VP4 protease during the assembly process are described in this example. The experimental design is based on the coexpression of the IBDV pVP2 and VP3 proteins from two independent chimeric genes. To that end, two recombinant baculoviruses (rBVs) described above, FB/his-VP3 (Kochan, G., González, D. & Rodríguez, J. F. (2003). Characterization of the RNA binding activity of VP3, a major structural protein of IBDV. Archives of Virology 148, 723-744) and FB/VPX [also identified as FB/pVP2 in this description] (Martínez-Torrecuadrada, J. L., Castón, J. R., Castro, M., Carrascosa, J. L., Rodríguez, J. F. & Casal, J. I. (2000). Different architectures in the assembly of infectious bursal disease virus capsid proteins expressed in insect cells. Virology 278, 322-331) have been used. These rBVs were generated by means of the cloning into suitable vectors of the complementary DNA (cDNA) encoders of the IBDV pVP2 and pVP3 proteins. Said cDNAs were obtained by RT-PCR from the A segment of the serotype I IBDV Soroa strain genome a (NCBI access number AAD30136). The rBV FB/his-VP3 expresses a chimeric VP3 protein which at its N-terminal end contains a tandem of six histidines fused to the VP3 sequence (Met754-G1u1012 of the polyprotein) called his-VP3. rBV FB/pVP2 expresses the encoding region of the pVP2 protein (Met1-Ala512).

The analysis of the expression of these pVP2 and his-pVP3 proteins, whether independently or together, was carried out in cell cultures. To carry out these experiments, single layer cell cultures from the insect *Trichloplusia ni* (H5, Invitrogen) were used, which were grown on cover glasses. Said cultures were independently infected with FB/pVP2, FB/his-VP3, or coinfected with both rBVs. The multiplicity of infection was 5 plaque forming units (pfu)/cell. The cells were fixed at 48 hours post-infection (h.p.i), and incubated with rabbit anti-VP2 polyclonal serum and with rat anti-VP3 polyclonal serum (Fernández-Arias, A., Risco, C., Martínez, S., Albar, J. P. & Rodríguez, J. F. (1998). Expression of ORF A1 of infectious bursal disease virus results in the formation of virus-like particles. Journal of General Virology 79, 1047-1054). After successive washings, the cover glasses were incubated with goat anti-rat serum conjugated with Alexa 594 and with goat anti-rabbit serum conjugated with Alexa 488 (Jackson Immunoresearch Laboratories, Inc.). The cellular cores were stained with the specific To-Pro-3 marker (Molecular Probes, Inc.). The samples were finally viewed by epifluorescence with a Zeiss Axiovert 200 microscope equipped with the Bio Rad Radiance 2100 confocal system. The images obtained were stored using the Laser Sharp Package (Bio Rad) software equipment. As is shown in Figure 2a, in the cultures infected with FB/pVP2, the anti-VP2 serum showed a fine granular signal mixed with tubular structures, both distributed throughout the cytoplasm. The anti-VP3 signal, detected in the cells infected with rBV FB/his-VP3, was characterized by the presence of spherical-shaped, and apparently hollow, accumulations around the core. In the cultures coinfected with both rBVs, a notable modification in the distribution pattern of both proteins was detected. In these cells, the specific signals of pVP2 and VP3 were collocated in spherical and dense accumulations, suggesting that their coexpression allowed the formation of pVP2/his-VP3 complexes (Figure 2c to 2e).

For the purpose of characterizing these structures in greater detail, similar extracts corresponding to cells infected with FB/pVP2+FB/hisVP3 were analyzed by transmission electron microscopy (TEM). As a control, and in parallel, H5 cell cultures infected with the wild strain of the FBD (FastBacDual, Invitrogen) virus were analyzed by the same technique. After the infection, the cells were harvested after 48 hours, and processed as has been previously described (Fernández-Arias A, Risco C, Martínez S, Albar JP & Rodríguez JF. (1998). Expression of ORF A1 of infectious bursal disease virus results in the formation of virus-like particles. Journal of General Virology 79:1047-1054) for their analysis in ultrathin sections by TRANSMISSION ELECTRON MICROSCOPY. As is shown in Figure 2, the cytoplasm of the coinfected cells contains aggregates formed by a mixture of tubules and structures similar to capsids (Figure 2g, 2h and 2i). These aggregates were not observed in any case in the samples corresponding to cells infected with wild FBD virus (Figure 2f). The appearance and size of the tubules, as well as of the structures similar to capsids, was similar to those previously described in cell cultures infected with VT7/Poly, a recombinant of the vaccinia virus expressing the gene of the IBDV polyprotein (Fernández-Arias A, Risco C, Martínez S, Albar JP & Rodríguez JF. (1998). Expression of ORF A1 of infectious bursal disease virus results in the formation of virus-like particles. Journal of General Virology 79:1047-1054).

To unmistakably establish that the coexpression of pVP2 and his-VP3 enabled the assembly and, therefore, the obtainment of VLPs-(VP4), the decision was made to purify the formed particles. To that end, H5 cell cultures were infected with FB/pVP2+FB/his-VP3. At 60 h.p.i., the cells were homogenized and the extracts were separated on sucrose gradients as previously described (Lombardo E, Maraver A, Castón JR, Rivera J, Fernández-Arias A, Serrano A, Carrascosa JL & Rodríguez JF. (1999). VP1, the putative RNA-dependent RNA polymerase of infectious bursal disease virus, forms complexes with the capsid protein VP3, leading to efficient encapsidation into virus-like particles. Journal of Virology 73:6973-6983). After their centrifugation, the gradients were fractioned, and the different fractions were analyzed by TEM as previously described (Lombardo and *et al.,* cited *supra*). As a control, and subject to the same process, gradients corresponding to cell extracts infected with rBV FB/VPX or with rBV FBD/Poly-VP1, were fractioned. The recombinant virus FBD/Poly-VP1 simultaneously expresses the VP1 polypeptide and polyprotein. As was predictable, the infection with FBD/Poly-VP1 had a result of an efficient production of VLPs (Maraver A, Oña A, Abaitua F, González D, Clemente R, Diaz-Ruiz A, Castón JR, Pazos F & Rodríguez JF. (2003). The oligomerization domain of VP3, the scaffolding protein of infectious bursal disease virus, plays a critical role for capsid formation. Journal of Virology 77:6438-49). On the other hand, the fractions corresponding to the cells infected with FB/VPX only contain tubules of a twisted appearance. The gradients corresponding to cells coinfected with the rBVs FB/pVP2+FB/his-VP3 contain rigid type I tubules in the fractions near the bottom of the gradient, and VLPs-(VP4) in the central and top fractions (Figure 3b). The VLPs-(VP4) isolated from the cells coinfected with rBV FB/pVP2+FB/his-VP3 had a diameter of 65-70 nm, as well as a typical polygonal contour, absolutely indistinguishable from the purified VLPs of cultures infected with FBD/Poly-VP1 (Maraver, A., Oña, A., Abaitua, F., González, D., Clemente, R., Diaz-Ruiz, A., Caston, J. R., Pazos, F. & Rodríguez, J. F. (2003). The oligomerization domain of VP3, the scaffolding protein of infectious bursal disease virus, plays a critical role for capsid formation. Journal of Virology 77:6438-49) or of the cultures infected with VT7/Poly (Fernández-Arias, A., Risco, C., Martínez, S., Albar, J. P. & Rodríguez, J. F. (1998). Expression of ORF A1 of infectious bursal disease virus results in the formation of virus-like particles. Journal of General Virology 79, 1047-1054).

For the purpose of achieving a biochemical characterization of the obtained material, Western blot experiments were carried out in which the different fractions were compared with specific sera against the VP1, pVP2, VP3 and VP4 proteins (Fernández-Arias *et al.* 1998, Lombardo *et al.,* 2000). Cell extracts infected with IBDV were used as a control. The obtained results are shown in Figure 3d. As was expected, the bands corresponding to the VP1 and VP4 polypeptides were only detected in samples corresponding to cells infected with FBD/Poly-VP1. The patterns corresponding to pVP2/VP3 in samples corresponding to cells infected with FBD/Poly-VP1 or coinfected with FB/VPX+ FB/his-VP3 were similar, two bands corresponding to pVP2 and VP3, respectively, being detected.

### 1.2 Obtaining VLPs(-VP4) by means of the coexpression of pVP2 (pVPX) and VP3 with an rBV in insect cells

Furthermore, the construction of the plasmid pFBD/pVP2-his-VP3 was carried out. The first step of the construction was carried out by means of the cloning of the encoding region of the pVP2 protein into the pFBDual vector (Invitrogen). The DNA fragment corresponding to pVP2 was obtained by means of PCR with the oligonucleotides identified as Oligo I (SEQ ID NO: 1) and Oligo II (SEQ ID NO: 2) using the plasmid pVOTE.2/Poly as a mold (Fernández-Arias, A., Risco, C., Martínez, S., Albar, J. P. & Rodríguez, J. F. (1998). Expression of ORF A1 of infectious bursal disease virus infectious results in the formation of virus-like particles. Journal of General Virology 79, 1047-1054). The fragment was purified, subjected to digestion with the BglII and HindIII enzymes and cloned into the pFBDual vector (Invitrogen) previously digested with the BamHI and Hindi enzymes. The resulting plasmid was called pFBD/pVP2. Then, a DNA fragment containing the open reading frame corresponding to the VP3 protein was obtained by means of digestion of the plasmid pFB/his-VP3 (Kochan et al., 2003, cited *supra*) with the RsrII enzyme, treatment with Klenow, and subsequent restriction with KpnI. This DNA fragment was purified and cloned into the plasmid pFBD/pVP2 previously digested with the SmaI and KpnI enzymes. The resulting plasmid was called pFBD/pVP2-his-VP3 (SEQ ID NO: 3) and contains the encoding nucleotide sequence of the pVP2 and his-pVP3 proteins (the latter is encoded by the complementary chain to the nucleotides 6734-7585 of SEQ ID NO: 3). The amino acid sequence of the pVP2 protein and of the his-VP3 fusion protein (pVP2-his-VP3) encoded by the nucleotide sequence contained in said plasmid pFBD/pVP2-his-VP3 is shown in SEQ ID NO: 4.

The plasmid pFBD/pVP2-his-VP3 allows obtaining an rBV, called FBD/pVP2-his-VP3, expressing both proteins simultaneously during its replication cycle [http://invitrogen.com/content/sfs/manuals/bevtest.pdf].

The results obtained with FBD/pVP2-his-VP3 are identical to those obtained by means of the coinfection with rBVs FB/pVP2 and FD/his-VP3, IBDV VLPs(-VP4) being obtained.

### EXAMPLE 2

### Obtaining VLPs(-VP4) by means of the coexpression of pVP2 and VP3 as two independent genes in yeasts

For the purpose of studying the possibility of obtaining IBDV VLPs(-VP4) in yeast cultures (*Saccharomyces cerevisiae*) the vector pESCURA/pVP2-VP3-GFP was generated with the heterologous GFP gene bound to the VP3 N-terminal end. The first step in the construction of the vector was carried out by means of the cloning of the encoding region of the IBDV pVP2 protein into the vector pESCURAinv. The plasmid pESCURAinv was generated by means of digestion of the vector pRS426 (Stratagene) with the PvuII enzyme and religation of the digestion mixture. The resulting vector, pESCURAinv, contains the multiple cloning region in reversed position with regard to that of parent vector pRS426. The DNA fragment corresponding to the pVP2 protein was obtained by means of PCR with the oligonucleotides called Oligo III (SEQ ID NO: 5) and Oligo IV (SEQ ID NO: 6) using the plasmid pVOTE.2/Poly as a mold (Fernández-Arias, A., Risco, C., Martínez, S., Albar, J. P. & Rodríguez, J. F. (1998). Expression of ORF A1 of infectious bursal disease virus results in the formation of virus-like particles. Journal of General Virology 79, 1047-1054). The fragment was purified subjected to digestion with the Bg1II and HindIII enzymes and cloned into the vector pESCURA.inv, previously digested with the BamHI and HindIII enzymes. The resulting plasmid was called pESCURA/pVP2.

The plasmid pFB/VP3-GFP was constructed in two stages. The first one consisted of the cloning of a DNA fragment, generated by means of PCR, containing the ORF of the IBDV VP3 protein lacking the termination codon. This PCR was carried out using the oligonucleotides called Oligo V (SEQ ID NO: 7) and Oligo VI (SEQ ID NO: 8) and using the plasmid pVOTE.2/Poly as a mold (Fernández-Arias, A., Risco, C., Martínez, S., Albar, J. P. & Rodríguez, J. F. (1998). Expression of ORF A1 of infectious bursal disease virus results in the formation of virus-like particles. Journal of General Virology 79, 1047-1054). The resulting DNA was digested with the EcoRI and BamHI enzymes and cloned into the vector pEGFP-N3 (Clontech), also digested with the same enzymes. The resulting plasmid was called pVP3-GFP. Then, the plasmid pEGFP-GFP was digested with the EcoRI and NotI enzymes and cloned into the vector pFastBac1 (Invitrogen). The resulting plasmid was called pFB/VP3-GFP.

Then, a DNA fragment that contained the open reading frame corresponding to the IBDV VP3 protein fused to the encoding region of the EGFP protein was obtained by means of digestion of the plasmid pFB/VP3-GFP with the EcoRI and NotI enzymes. This DNA fragment was purified and cloned into the plasmid pESCUR.A/pVP2 previously digested with the EcoRI and NotI enzymes. The resulting plasmid was called pESCURA/pVP2-VP3-GFP (SEQ ID NO: 9) and contains the ORFs of the pVP2 and VP3-GFP proteins under the transcriptional control of two independent promoters, GAL 1 and GAL 10, both inducible by galactose (the pVP2 protein is encoded by the chain of nucleotides complementary to the nucleotides 5862-7343 of SEQ ID NO: 9). The amino acid sequence of the pVP2 protein and of the VP3-GFP fusion protein (pVP2-VP3-GFP) encoded by the nucleotide sequence contained in said plasmid pESCURA/pVP2-VP3-GFP is shown in SEQ ID NO: 10.

pESCURA/pVP2-VP3-GFP was subsequently used to transform a culture of *S. cerevisiae* yeast haploid strain 499 according to a previously described protocol (Gietz, R.D. and R.A. Woods. (2002), Transformation of yeast by the Liac/SS carrier DNA/PEG method. Methods in Enzymology 350:87-96). The yeasts transformed with the plasmid were selected by means of growth on SC medium plates (CSM + YNB, 2% glucose and bacto agar) supplemented with the amino acids tryptophan, leucine and histidine and lacking uracyl (-Ura). After an incubation of 48 hours at 30°C, a colony was chosen which was used to carry out the following protein expression and VLPs-(VP4) formation analyses.

The pVP2 and VP3 protein expression and VLPs-(VP4) formation analyses were carried out following a protocol previously described for the characterization of IBDV VLPs in other expression systems (Fernández-Arias, A., Risco, C., Martínez, S., Albar, J. P. & Rodríguez, J. F. (1998). Expression of ORF A1 of infectious bursal disease virus results in the formation of virus-like particles. Journal of General Virology 79, 1047-1054; Lombardo, E., Maraver, A., Castón, J. R., Rivera, J., Fernández-Arias, A., Serrano, A., Carrascosa, J. L. & Rodríguez, J. F. (1999). VP1, the putative RNA-dependent RNA polymerase of infectious bursal disease virus, forms complexes with the capsid protein VP3, leading to efficient encapsidation into virus-like particles. Journal of Virology 73, 6973-698). The colony selected was cultured in liquid CSM (-Ura) + YNB medium supplemented with 2% raffinose. The culture was incubated at 30°C for 24 hours. This culture was used to inoculate, at an optical density (O.D.) of 0.2, a flask of 200 ml of CSM (-Ura) + YNB medium supplemented with 2% inducer galactose. The culture was maintained at 30°C for 18 hours (until an O.D. between 1.0 and 2.0). The yeasts were centrifuged at 3,000 radiant power measurement, 5 minutes at 4°C, were washed once with distilled water, and the pellet was resuspended in lysis buffer (TEN: Tris 10 mM, pH 8.0; NaCl 150 mM; EDTA 1 mM) + 2X protease inhibitors (Compl Roche). A volume of glass beads having a size of about 425-600 microns (Sigma) were added for the lysis. This mixture was subjected to vigorous vortex stirring for 30 seconds 4 times, with 30-second intervals, and at 4°C. After this, the soluble fraction was recovered by centrifuging the lysis mixture at 13,000 rpm for 15 minutes at 4°C. This sample was subjected to fractioning on a sucrose gradient according to a previously described protocol (Lombardo, E., Maraver, A., Castón, J. R., Rivera, J., Fernández-Arias, A., Serrano, A., Carrascosa, J. L. & Rodríguez, J. F. (1999). VP1, the putative RNA-dependent RNA polymerase of infectious bursal disease virus, forms complexes with the capsid protein VP3, leading to efficient encapsidation into virus-like particles. Journal of Virology 73, 6973-6983). The samples obtained after fractioning as well as a sample of the starting material were analyzed by means of sodium dodecylsulfate polyacrylamide gel electrophoresis (SDS-PAGE) [Current Protocols in Molecular Biology] and immunodetection by Western blot (Figure 4A) using anti-pVP2 and anti-VP3 sera [Current Protocols in Molecular Biology]. As is shown in Figure 4A, the Western blot showed the presence of bands, with the predicted molecular mass corresponding to the pVP2 (48 kDa) and VP3-GFP (61 kDa) proteins, as well as other immunoreactive bands of a smaller size probably produced by proteolytic degradation both in the initial sample and in the different fractions of the gradient. These results reliably showed the correct expression of both polypeptides in the *S. cerevisiae* culture transformed with the plasmid pESCURA/pVP2-VP3. Then, the different fractions of the gradient were analyzed by means of TEM as has been previously described (Lombardo, E., Maraver, A., Castón, J. R., Rivera, J., Fernández-Arias, A., Serrano, A., Carrascosa, J. L. & Rodríguez, J. F. (1999). VP1, the putative RNA-dependent RNA polymerase of infectious bursal disease virus, forms complexes with the capsid protein VP3, leading to efficient encapsidation into virus-like particles. Journal of Virology 73, 6973-6983). As is shown in Figure 4B, the TEM analysis of the fractions of the gradient showed the existence of IBDV VLPs in the top fractions of the gradient. These VLPs, VLPs(-VP4), have a diameter of 65-70 nm and a polygonal contour that is indistinguishable from the IBDV VLPs obtained in other expression systems (Figure 4C).

## Claims

1. An empty capsid of the infectious bursal disease virus (IBDV), VLP(-VP4), **characterized in that** it is constituted by assembly of only IBDV pVP2 proteins and IBDV VP3 proteins.

2. A nucleic acid **characterized in that** its nucleotide sequence is constituted by (i) a nucleotide sequence consisting of the open reading frame corresponding to the IBDV pVP2 protein and (ii) a nucleotide sequence consisting of the open reading frame corresponding to the IBDV VP3 protein.

3. A gene construct comprising a nucleic acid according to claim 2.

4. An expression system selected from:
a) an expression system consisting of (i) a gene construct consisting of the open reading frame corresponding to the IBDV pVP2 protein, operatively bound to transcription, and optionally translation, control elements, and (ii) a gene construct consisting of the open reading frame corresponding to the IBDV VP3 protein, operatively bound to transcription, and optionally translation, control elements; and
b) an expression system consisting of a gene construct according to claim 3, operatively bound to transcription, and optionally translation, control elements.

5. An expression system according to claim 4, said expression system being selected from plasmids, bacmids, yeast artificial chromosomes (YACs), bacteria artificial chromosomes (BACs), bacteriophage P1-based artificial chromosomes (PACs), cosmids, and viruses, which, optionally, contain a heterologous replication origin.

6. A host cell containing a nucleic acid according to claim 2, or a gene construct according to claim 3, or an expression system according to anyone of claims 4 or 5.

7. A host cell that is transformed, transfected or infected with an expression system according to anyone of claims 4 or 5.

8. Host cell according to anyone of claims 6 or 7, said cell being an insect cell or a yeast.

9. A process for the production of empty capsids of the infectious bursal disease virus (IBDV), VLPs(-VP4), according to claim 1, comprising culturing a host cell according to anyone of claims 6 to 8, and if so desired, recovering said empty IBDV capsids.

10. Process according to claim 9, wherein said host cell is an insect cell, comprising the steps of:
a) preparing an expression system selected from:
- an expression system constituted by a recombinant baculovirus containing a gene construct according to claim 3, operatively bound to transcription, and optionally translation, control elements; and
- an expression system constituted by (i) a recombinant baculovirus containing a gene construct comprising the open reading frame corresponding to the IBDV pVP2 protein, and (ii) a recombinant baculovirus containing a gene construct comprising the open reading frame corresponding to the IBDV VP3 protein;
b) infecting insect cells with said expression system prepared in step a);
c) culturing the infected insect cells obtained in step b) under conditions allowing the expression of recombinant proteins and their assembly for forming empty IBDV capsids, VLPs(-VP4); and
d) if so desired, isolating and optionally purifying said IBDV empty capsids, VLPs(-VP4).

11. Process according to claim 9, wherein said host cell is a yeast, comprising the steps of:
a) preparing an expression system constituted by a plasmid containing a gene construct according to claim 3;
b) transforming yeast cells with said expression system prepared in step a);
c) culturing the transformed yeasts obtained in step b) under conditions allowing the expression of recombinant proteins and their assembly to form empty IBDV capsids, VLPs(-VP4); and
d) if so desired, isolating and optionally purifying the empty IBDV capsids, VLPs(-VP4).

12. The use of a gene expression system according to anyone of claims 4 or 5 for producing and obtaining empty IBDV capsids, VLPs(-VP4), according to claim 1.

13. The use of empty capsids of the infectious bursal disease virus (IBDV), VLPs(-VP4), according to claim 1 in the manufacture of a medicament.

14. Use according to claim 13, wherein said medicament is a vaccine against the avian disease called infectious bursal disease.

15. Use according to claim 13, wherein said medicament is a gene therapy vector.

16. A vaccine comprising a therapeutically effective amount of empty IBDV capsids, VLPs(-VP4), according to claim 1, optionally together with one or more pharmaceutically acceptable adjuvants and/or vehicles.

17. Vaccine according to claim 16 to protect birds from the infection caused by the infectious bursal disease virus (IBDV).

18. Vaccine according to claim 17, wherein said birds are selected from the group formed by chickens, turkeys, geese, ganders, pheasants, quails and ostriches.

19. Vaccine to protect chickens from the infection caused by the infectious bursal disease virus (IBDV) comprising a therapeutically effective amount of empty IBDV capsids, VLPs(-VP4), according to claim 1, optionally together with one or more pharmaceutically acceptable adjuvants and/or vehicles.

## Patentansprüche

1. Leeres Kapsid des Virus der infektiösen Bursitis (IBDV), VLP(-VP4), **dadurch gekennzeichnet, dass** es ausschließlich durch die Verbindung von Proteinen pVP2 des IBDV und Proteinen VP3 des IBDV gebildet wird.

2. Nukleinsäure **dadurch gekennzeichnet, dass** ihre Nukleotidsequenz aus (i) einer Nukleotidsequenz, bestehend aus dem offenen Leserahmen entsprechend dem Protein pVP2 des IBDV und (ii) einer Nukleotidsequenz, bestehend aus dem offenen Leserahmen entsprechend dem Protein VP3 des IBDV besteht.

3. Genetisches Konstrukt, das eine Nukleinsäure gemäß Anspruch 2 umfasst.

4. Expressionssystem ausgewählt aus:
a) einem Expressionssystem bestehend aus (i) einem genetischen Konstrukt, bestehend aus dem offenen Leserahmen entsprechend dem Protein pVP2 des IBDV, funktionell verbunden mit Transkriptions-, und gegebenenfalls mit Translationskontrollelementen, und (ii) einem genetischen Konstrukt, bestehend aus dem offenen Leserahmen entsprechend dem Protein VP3 des IBDV, funktionell verbunden mit Transkriptions-, und gegebenenfalls mit Translationskontrollelementen; und
b) einem Expressionssystem bestehend aus einem genetischen Konstrukt gemäß Anspruch 3, funktionell verbunden mit Transkriptions-, und gegebenenfalls mit Translationskontrollelementen.

5. Expressionssystem gemäß Anspruch 4, wobei dieses Expressionssystem aus Plasmiden, Bacmiden, künstlichen Hefechromosomen (YACs), künstlichen Bakterienchromosomen (BACs), künstlichen Chromosomen basierend auf der Bakteriophage P1 (PACs), Cosmiden, und Viren ausgewählt wird, die gegebenenfalls einen heterologen Replikationsursprung enthalten.

6. Wirtszelle die eine Nukleinsäure gemäß Anspruch 2 oder ein genetisches Konstrukt gemäß Anspruch 3, oder ein Expressionssystem gemäß einem der Ansprüche 4 oder 5 enthält.

7. Wirtszelle die mit einem Expressionssystem gemäß einem der Ansprüche 4 oder 5 transformiert, transfiziert oder infiziert ist.

8. Wirtszelle gemäß einem der Ansprüchen 6 oder 7, wobei diese Zelle eine Insektenzelle oder eine Hefe ist.

9. Verfahren zur Herstellung von leeren Kapsiden des Virus der infektiösen Bursitis (IBDV), (VLP)s(-VLP4), gemäß Anspruch 1, das die Kultivierung einer Wirtszelle gemäß einem der Ansprüche 6 bis 8, und falls gewünscht, die Rückgewinnung dieser leeren Kapsiden des IBDV umfasst.

10. Verfahren gemäß Anspruch 9, wobei diese Wirtszelle eine Insektenzelle ist, das die folgenden Schritte umfasst:
a) Aufbau eines Expressionssystem ausgewählt aus:
- einem Expressionssystem bestehend aus einem rekombinanten Baculovirus das ein genetisches Konstrukt gemäß Anspruch 3 enthält, funktionell verbunden mit Transkriptions-, und gegebenenfalls mit Translationskontrollelementen; und
- einem Expressionssystem bestehend aus (i) einem rekombinanten Baculovirus das ein genetisches Konstrukt enthält, das den offenen Leserahmen entsprechend dem Protein pVP2 des IBDV umfasst, und (ii) einem rekombinanten Baculovirus, das ein genetisches Konstrukt enthält, das den offenen Leserahmen entsprechend dem Protein VP3 des IBDV umfasst;
b) Infektion von Insektenzellen mit diesem, im Schritt a) aufgebauten Expressionssystems;
c) Kultivierung der in Schritt b) erhaltenen infizierten Insektenzellen unter Bedingungen die die Expression von rekombinanten Proteinen und ihre Verbindung zur Bildung von leeren Kapsiden des IBDV, VLPs(-VP4), erlauben; und
d) falls gewünscht, Isolierung und gegebenenfalls Reinigung dieser leeren Kapside des IBDV, VLPs(-VP4).

11. Verfahren gemäß Anspruch 9, in dem diese Wirtszelle eine Hefe ist, das die folgenden Schritte umfasst:
a) Aufbau eines Expressionssystem bestehend aus einem Plasmid, das ein genetisches Konstrukt gemäß Anspruch 3 enthält;
b) Transformation von Hefezellen mit diesem in Schritt a) aufgebauten Expressionssystems;
c) Kultivierung von transformierten, im Schritt b) erhaltenen Hefen unter Bedingungen die die Expression von rekombinanten Proteinen und ihre Verbindung zur Bildung von leeren Kapsiden des IBDV, VLPs(-VP4), erlauben; und
d) falls gewünscht, Isolierung und gegebenenfalls Reinigung der leeren Kapside des IBDV, VLPs(-VP4).

12. Verwendung eines Gen-Expressionssystem gemäß einem der Ansprüche 4 oder 5, zur Herstellung und Gewinnung leerer Kapsiden des IBDV, VLPs(-VP4), gemäß Anspruch 1.

13. Verwendung von leeren Kapsiden des Virus der infektiösen Bursitis (IBDV), VLPs(-VP4), gemäß Anspruch 1, in der Produktion eines Medikamentes.

14. Verwendung gemäß Anspruch 13, wobei dieses Medikament ein Impfstoff gegen die als infektiöse Bursitis bezeichnete Vogelkrankheit.

15. Verwendung gemäß Anspruch 13, wobei dieses Medikament ein Gentherapievektor ist.

16. Impfstoff, der eine therapeutisch effiziente Dosis leerer Kapside des IBDV, VLPs(-VP4), umfasst, gemäß Anspruch 1, gegebenenfalls zusammen mit einem oder mehreren pharmazeutisch akzeptablen Hilfsmittel und/oder Trägersubstanzen.

17. Impfstoff gemäß Anspruch 16, zum Schutz von Vögeln vor der durch den Virus der infektiösen Bursitis (IBDV) verursachten Infektion.

18. Impfstoff gemäß Anspruch 17, wobei diese Vögel aus der Gruppe der Hühner, Truthähne, Enten, Gänse, Fasane, Wachteln und Straussen ausgewählt werden.

19. Impfstoff zum Schutz der Hühner vor der durch den Virus der infektiösen Bursitis (IBDV) verursachten Infektion, der eine therapeutisch effiziente Menge leerer Kapsiden des IBDV, VLPs(-VP4), umfasst, gemäß Anspruch 1, gegebenenfalls zusammen mit einem oder mehreren pharmazeutisch akzeptablen Hilfsmittel und/oder Trägersubstanzen.

## Revendications

1. Capside vide du virus de la bursite infectieuse (IBDV), VLP(-VP4), **caractérisé en ce qu'**elle est constituée par l'union seulement des protéines pVP2 d'IBDV et des protéines VP3 d'IBDV.

2. Acide nucléique **caractérisé en ce que** sa séquence de nucléotides est constituée par (i) une séquence de nucléotides consistant en le cadre de lecture ouvert correspondant à la protéine pVP2 d'IBDV et (ii) une séquence de nucléotides consistant en le cadre de lecture ouvert correspondant à la protéine VP3 d'IBDV.

3. Construction génique comprenant un acide nucléique selon la revendication 2.

4. Système d'expression sélectionné parmi:
a) un système d'expression consistant en (i) une construction génique consistant en le cadre de lecture ouvert correspondant à la protéine pVP2 d'IBDV, fonctionnellement unie à des éléments de contrôle de transcription, et optionnellement de traduction, et (ii) une construction génique consistant en le cadre de lecture ouvert correspondant à la protéine VP3 d'IBDV, fonctionnellement unie à des éléments de contrôle de transcription, et optionnellement de traduction; et
b) un système d'expression consistant en une construction génique selon la revendication 3, fonctionnellement uni à des éléments de contrôle de transcription, et optionnellement traduction.

5. Système d'expression selon la revendication 4, ledit système d'expression étant sélectionné parmi des plasmides, bacmides, chromosomes artificiels de levure (YAC), chromosomes artificiels de bactéries (BAC), chromosomes artificiels basés sur des bactériophages P1 (PAC), cosmides, et virus, qui contiennent optionnellement une origine de réplication hétérologue.

6. Cellule hôte contenant un acide nucléique selon la revendication 2, o une construction génique selon la revendication 3, ou un système d'expression selon l'une quelconque des revendications 4 ou 5.

7. Cellule hôte qui est transformée, transfectée ou infectée avec un système d'expression selon l'une quelconque des revendications 4 ou 5.

8. Cellule hôte selon l'une quelconque des revendications 6 ou 7, ladite cellule étant une cellule d'insecte ou une levure.

9. Procédé pour la production de capsides vides du virus de la bursite infectieuse (IBDV), VLP(-VP4), selon la revendication 1, comprenant la culture d'une cellule hôte selon l'une quelconque des revendications 6 à 8, et si l'on le souhaite, la récupération desdites capsides d'IBDV vides.

10. Procédé selon la revendication 9, dans lequel ladite cellule hôte est une cellule d'insecte, comprenant les étapes de:
a) préparer un système d'expression sélectionné parmi:
- un système d'expression constitué par un baculovirus recombinant contenant une construction génique selon la revendication 3, fonctionnellement uni à des éléments de contrôle de transcription, et optionnellement de traduction; et
- un système d'expression constitué par (i) un baculovirus recombinant contenant une construction génique comprenant le cadre de lecture ouvert correspondant à la protéine pVP2 d'IBDV, et (ii) un baculovirus recombinant contenant une construction génique comprenant le cadre de lecture ouvert correspondant à la protéine VP3 d'IBDV;
b) infecter des cellules d'insecte avec ledit système d'expression préparé lors de l'étape a);
c) faire une culture des cellules d'insecte infectées obtenues lors de l'étape b) sous des conditions permettant l'expression de protéines recombinantes et leur union pour former des capsides d'IBDV vides, VLP(-VP4); et
d) si l'on le souhaite, isoler et optionnellement purifier lesdites capsides vides de IBDV, VLP(-VP4).

11. Procédé selon la revendication 9, dans lequel ladite cellule hôte est une levure, comprenant les étapes de:
a) préparer un système d'expression constitué par un plasmide contenant une construction génique selon la revendication 3;
b) transformer des cellules de levure avec ledit système d'expression préparé lors de l'étape a);
c) faire une culture des levures transformées obtenues lors de l'étape b) sous des conditions permettant l'expression de protéines recombinantes et leur union pour former des capsides d'IBDV vides, VLP(-VP4); et
d) si l'on le souhaite, isoler et optionnellement purifier les capsides d'IBDV vides, VLP(-VP4).

12. Utilisation d'un système d'expression génique selon l'une quelconque des revendications 4 ou 5, pour produire et obtenir des capsides d'IBDV vides, VLP(-VP4), selon la revendication 1.

13. Utilisation de capsides vides du virus de la bursite infectieuse (IBDV), VLP(-VP4), selon la revendication 1, dans la fabrication d'un médicament.

14. Utilisation selon la revendication 13, dans laquelle ledit médicament est un vaccin contre la maladie aviaire dite bursite infectieuse.

15. Utilisation selon la revendication 13, dans laquelle ledit médicament est un vecteur de traitement génique.

16. Vaccin comprenant une quantité thérapeutiquement efficace de capsides d'IBDV vides, VLP(-VP4), selon la revendication 1, optionnellement avec un ou plusieurs véhicules et/ou adjuvants pharmaceutiquement acceptables.

17. Vaccin selon la revendication 16, pour protéger les oiseaux contre l'infection produite par le virus de la bursite infectieuse (IBDV).

18. Vaccin selon la revendication 17, dans lequel lesdits oiseux sont sélectionnés parmi le groupe formé par les poulets, les dindes, les oies, les jars, les faisans, les cailles et les autruches.

19. Vaccin pour protéger les poulets contre l'infection produite par le virus de la bursite infectieuse (IBDV) comprenant une quantité thérapeutiquement efficace de capsides d'IBDV vides, VLP(-VP4), selon la revendication 1, optionnellement avec un ou plusieurs véhicules et/ou adjuvants pharmaceutiquement acceptables.


### SEQUENCE LISTING

<110> CONSEJO SUPERIOR DE INVESTIGACIONES CIENTIFICAS
   <110> BIONOSTRA, S.L.
<120> EMPTY CAPSIDS (VLPs(-VP4)) OF THE INFECTIOUS BURSAL DISEASE VIRUS (IBDV), OBTAINMENT PROCESS AND APPLICATIONS
<130> P1392PC
<150> ES P200400121
   <151> 2004-01-21 (January 21, 2004)
<160> 10
   <170> PatentIn version 3.1
<210> 1
   <211> 35
   <212> DNA
   <213> Artificial sequence
<220> Synthetic DNA
   <223> Oligo I primer
<400> 1
   gcgcagatct atgacaaacc tgtcagatca aaccc 35
<210> 2
   <211> 34
   <212> DNA
   <213> Artificial sequence
<220> Synthetic DNA
   <223> Oligo II primer
<400> 2
   gcgcaagctt aggcgagagt cagctgcctt atgc 34
<210> 3
   <211> 7595
   <212> DNA
   <213> Artificial sequence
<220>
   <223> Plasmid pFBD/pVP2-his-VP3
<221> promoter
   <222> (157)..(285)
   <223> Promotor ppolh
<221> CDS
   <222> (291)..(1289)
   <223> pVP2 ORF
<221> promoter
   <222> (7443)..(7503)
   <223> Promoter p10
<400> 3
   gggtgatcaa gtcttcgtcg agtgattgta aataaaatgt aatttacagt atagtatttt      60
   aattaatata caaatgattt gataataatt cttatttaac tataatatat tgtgttgggt     120
   tgaattaaag gtccgtatac tccggaatat taatagatca tggagataat taaaatgata     180
   accatctcgc aaataaataa gtattttact gttttcgtaa cagttttgta ataaaaaaac     240
   ctataaatat tccggattat tcataccgtc ccaccatcgg gcgcggatct atg aca        296
                                                          Met Thr
                                                          1
   aac ctg tca gat caa acc cag cag att gtt ccg ttc ata cgg agc ctt       344
   Asn Leu Ser Asp Gln Thr Gln Gln Ile Val Pro Phe Ile Arg Ser Leu
           5                   10                  15
   ctg atg cca aca acc gga ccg gcg tcc att ccg gac gac acc ctg gag       392
   Leu Met Pro Thr Thr Gly Pro Ala Ser Ile Pro Asp Asp Thr Leu Glu
       20                  25                  30
   aag cac act ctc agg tca gag acc tcg acc tac aat ttg act gtg ggg       440
   Lys His Thr Leu Arg Ser Glu Thr Ser Thr Tyr Asn Leu Thr Val Gly
   35                  40                  45                  50
   gac aca ggg tca ggg cta att gtc ttt ttc cct gga ttc cct ggc tca       488
   Asp Thr Gly Ser Gly Leu Ile Val Phe Phe Pro Gly Phe Pro Gly Ser
                   55                  60                  65
   att gtg ggt gct cac tac aca ctg cag ggc aat ggg aac tac aag ttc       536
   Ile Val Gly Ala His Tyr Thr Leu Gln Gly Asn Gly Asn Tyr Lys Phe
               70                  75                  80
   gat cag atg ctc ctg act gcc cag aac cta ccg gcc agt tac aac tac       584
   Asp Gln Met Leu Leu Thr Ala Gln Asn Leu Pro Ala Ser Tyr Asn Tyr
           85                  90                  95
   tgc agg cta gtg agt cgg agt ctc aca gtg agg tca agc aca ctt cct       632
   Cys Arg Leu Val Ser Arg Ser Leu Thr Val Arg Ser Ser Thr Leu Pro
       100                 105                 110
   ggt ggc gtt tat gca cta aac ggc acc ata aac gcc gtg acc ttc caa       680
   Gly Gly Val Tyr Ala Leu Asn Gly Thr Ile Asn Ala Val Thr Phe Gln
   115                 120                 125                 130
   gga agc ctg agt gaa ctg aca gat gtt agc tac aat ggg ttg atg tct       728
   Gly Ser Leu Ser Glu Leu Thr Asp Val Ser Tyr Asn Gly Leu Met Ser
                   135                 140                 145
   gca aca gcc aac atc aac gac aaa att ggg aac gtc cta gta ggg gaa       776
   Ala Thr Ala Asn Ile Asn Asp Lys Ile Gly Asn Val Leu Val Gly Glu
               150                 155                 160
   ggg gtc acc gtc ctc agc tta ccc aca tca tat gat ctt ggg tat gtg       824
   Gly Val Thr Val Leu Ser Leu Pro Thr Ser Tyr Asp Leu Gly Tyr Val
           165                 170                 175
   agg ctt ggt gac ccc att ccc gca ata ggg ctt gac cca aaa atg gta      872
   Arg Leu Gly Asp Pro Ile Pro Ala Ile Gly Leu Asp Pro Lys Met Val
       180                 185                 190
   gcc aca tgt gac agc agt gac agg ccc aga gtc tac acc ata act gca      920
   Ala Thr Cys Asp Ser Ser Asp Arg Pro Arg Val Tyr Thr Ile Thr Ala
   195                 200                 205                 210
   gcc gat gat tac caa ttc tca tca cag tac caa cca ggt ggg gta aca      968
   Ala Asp Asp Tyr Gln Phe Ser Ser Gln Tyr Gln Pro Gly Gly Val Thr
                   215                 220                 225
   atc aca ctg ttc tca gcc aac att gat gcc atc aca agc ctc agc gtt     1016
   Ile Thr Leu Phe Ser Ala Asn Ile Asp Ala Ile Thr Ser Leu Ser Val
               230                 235                 240
   ggg gga gag ctc gtg ttt cga aca agc gtc cac ggc ctt gta ctg ggc     1064
   Gly Gly Glu Leu Val Phe Arg Thr Ser Val His Gly Leu Val Leu Gly
           245                 250                 255
   gcc acc atc tac ctc ata ggc ttt gat ggg aca acg gta atc acc agg     1112
   Ala Thr Ile Tyr Leu Ile Gly Phe Asp Gly Thr Thr Val Ile Thr Arg
       260                 265                 270
   gct gtg gcc gca aac aat ggg ctg acg acc ggc acc gac aac ctt atg     1160
   Ala Val Ala Ala Asn Asn Gly Leu Thr Thr Gly Thr Asp Asn Leu Met
   275                 280                 285                 290
   cca ttc aat ctt gtg att cca aca aac gag ata acc cag cca atc aca     1208
   Pro Phe Asn Leu Val Ile Pro Thr Asn Glu Ile Thr Gln Pro Ile Thr
                   295                 300                 305
   tcc atc aaa ctg gag ata gtg acc tcc aaa agt ggt ggt cag gca ggg     1256
   Ser Ile Lys Leu Glu Ile Val Thr Ser Lys Ser Gly Gly Gln Ala Gly
               310                 315                 320
   gat cag atg tca tgg tcg gca aga ggg agc cta gcagtgacga tccatggtgg   1309
   Asp Gln Met Ser Trp Ser Ala Arg Gly Ser Leu
           325                 330
   caactatcca ggggccctcc gtcccgtcac gctagtggcc tacgaaagag tggcaacagg   1369
   atccgtcgtt acggtcgctg gggtgagcaa cttcgagctg atcccaaatc ctgaactagc    1429
   aaagaacctg gttacagaat acggccgatt tgacccagga gccatgaact acacaaaatt   1489
   gatactgagt gagagggacc gtcttggcat caagaccgtc tggccaacaa gggagtacac   1549
   tgactttcgt gaatacttca tggaggtggc cgacctcaac tctcccctga agattgcagg   1609
   agcattcggc ttcaaagaca taatccgggc cataaggagg atagctgtgc cggtggtctc   1669
   cacattgttc ccacctgccg ctcccctagc ccatgcaatt ggggaaggtg tagactacct   1729
   gctgggcgat gaggcccagg ccgcttcagg aactgctcga gccgcgtcag gaaaagcaag   1789
   agctgcctca ggccgcataa ggcagctgac tctcgcctaa gcttgtcgag aagtactaga   1849
   ggatcataat cagccatacc acatttgtag aggttttact tgctttaaaa aacctcccac   1909
   acctccccct gaacctgaaa cataaaatga atgcaattgt tgttgttaac ttgtttattg 1969
   cagcttataa tggttacaaa taaagcaata gcatcacaaa tttcacaaat aaagcatttt 2029
   tttcactgca ttctagttgt ggtttgtcca aactcatcaa tgtatcttat catgtctgga 2089
   tctgatcact gcttgagcct aggagatccg aaccagataa gtgaaatcta gttccaaact 2149
   attttgtcat ttttaatttt cgtattagct tacgacgcta cacccagttc ccatctattt 2209
   tgtcactctt ccctaaataa tccttaaaaa ctccatttcc acccctccca gttcccaact 2269
   attttgtccg cccacagcgg ggcatttttc ttcctgttat gtttttaatc aaacatcctg 2329
   ccaactccat gtgacaaacc gtcatcttcg gctacttttt ctctgtcaca gaatgaaaat 2389
   ttttctgtca tctcttcgtt attaatgttt gtaattgact gaatatcaac gcttatttgc 2449
   agcctgaatg gcgaatggga cgcgccctgt agcggcgcat taagcgcggc gggtgtggtg 2509
   gttacgcgca gcgtgaccgc tacacttgcc agcgccctag cgcccgctcc tttcgctttc 2569
   ttcccttcct ttctcgccac gttcgccggc tttccccgtc aagctctaaa tcgggggctc 2629
   cctttagggt tccgatttag tgctttacgg cacctcgacc ccaaaaaact tgattagggt 2689
   gatggttcac gtagtgggcc atcgccctga tagacggttt ttcgcccttt gacgttggag 2749
   tccacgttct ttaatagtgg actcttgttc caaactggaa caacactcaa ccctatctcg 2809
   gtctattctt ttgatttata agggattttg ccgatttcgg cctattggtt aaaaaatgag 2869
   ctgatttaac aaaaatttaa cgcgaatttt aacaaaatat taacgtttac aatttcaggt 2929
   ggcacttttc ggggaaatgt gcgcggaacc cctatttgtt tatttttcta aatacattca 2989
   aatatgtatc cgctcatgag acaataaccc tgataaatgc ttcaataata ttgaaaaagg 3049
   aagagtatga gtattcaaca tttccgtgtc gcccttattc ccttttttgc ggcattttgc 3109
   cttcctgttt ttgctcaccc agaaacgctg gtgaaagtaa aagatgctga agatcagttg 3169
   ggtgcacgag tgggttacat cgaactggat ctcaacagcg gtaagatcct tgagagtttt 3229
   cgccccgaag aacgttttcc aatgatgagc acttttaaag ttctgctatg tggcgcggta 3289
   ttatcccgta ttgacgccgg gcaagagcaa ctcggtcgcc gcatacacta ttctcagaat 3349
   gacttggttg agtactcacc agtcacagaa aagcatctta cggatggcat gacagtaaga 3409
   gaattatgca gtgctgccat aaccatgagt gataacactg cggccaactt acttctgaca 3469
   acgatcggag gaccgaagga gctaaccgct tttttgcaca acatggggga tcatgtaact 3529
   cgccttgatc gttgggaacc ggagctgaat gaagccatac caaacgacga gcgtgacacc 3589
   acgatgcctg tagcaatggc aacaacgttg cgcaaactat taactggcga actacttact 3649
   ctagcttccc ggcaacaatt aatagactgg atggaggcgg ataaagttgc aggaccactt 3709
   ctgcgctcgg cccttccggc tggctggttt attgctgata aatctggagc cggtgagcgt 3769
   gggtctcgcg gtatcattgc agcactgggg ccagatggta agccctcccg tatcgtagtt 3829
   atctacacga cggggagtca ggcaactatg gatgaacgaa atagacagat cgctgagata 3889
   ggtgcctcac tgattaagca ttggtaactg tcagaccaag tttactcata tatactttag 3949
   attgatttaa aacttcattt ttaatttaaa aggatctagg tgaagatcct ttttgataat 4009
   ctcatgacca aaatccctta acgtgagttt tcgttccact gagcgtcaga ccccgtagaa 4069
   aagatcaaag gatcttcttg agatcctttt tttctgcgcg taatctgctg cttgcaaaca 4129
   aaaaaaccac cgctaccagc ggtggtttgt ttgccggatc aagagctacc aactcttttt 4189
   ccgaaggtaa ctggcttcag cagagcgcag ataccaaata ctgtccttct agtgtagccg 4249
   tagttaggcc accacttcaa gaactctgta gcaccgccta catacctcgc tctgctaatc 4309
   ctgttaccag tggctgctgc cagtggcgat aagtcgtgtc ttaccgggtt ggactcaaga 4369
   cgatagttac cggataaggc gcagcggtcg ggctgaacgg ggggttcgtg cacacagccc 4429
   agcttggagc gaacgaccta caccgaactg agatacctac agcgtgagca ttgagaaagc 4489
   gccacgcttc ccgaagggag aaaggcggac aggtatccgg taagcggcag ggtcggaaca 4549
   ggagagcgca cgagggagct tccaggggga aacgcctggt atctttatag tcctgtcggg 4609
   tttcgccacc tctgacttga gcgtcgattt ttgtgatgct cgtcaggggg gcggagccta 4669
   tggaaaaacg ccagcaacgc ggccttttta cggttcctgg ccttttgctg gccttttgct 4729
   cacatgttct ttcctgcgtt atcccctgat tctgtggata accgtattac cgcctttgag 4789
   tgagctgata ccgctcgccg cagccgaacg accgagcgca gcgagtcagt gagcgaggaa 4849
   gcggaagagc gcctgatgcg gtattttctc cttacgcatc tgtgcggtat ttcacaccgc 4909
   agaccagccg cgtaacctgg caaaatcggt tacggttgag taataaatgg atgccctgcg 4969
   taagcgggtg tgggcggaca ataaagtctt aaactgaaca aaatagatct aaactatgac 5029
   aataaagtct taaactagac agaatagttg taaactgaaa tcagtccagt tatgctgtga 5089
   aaaagcatac tggacttttg ttatggctaa agcaaactct tcattttctg aagtgcaaat 5149
   tgcccgtcgt attaaagagg ggcgtggcca agggcatggt aaagactata ttcgcggcgt 5209
   tgtgacaatt taccgaacaa ctccgcggcc gggaagccga tctcggcttg aacgaattgt 5269
   taggtggcgg tacttgggtc gatatcaaag tgcatcactt cttcccgtat gcccaacttt 5329
   gtatagagag ccactgcggg atcgtcaccg taatctgctt gcacgtagat cacataagca 5389
   ccaagcgcgt tggcctcatg cttgaggaga ttgatgagcg cggtggcaat gccctgcctc 5449
   cggtgctcgc cggagactgc gagatcatag atatagatct cactacgcgg ctgctcaaac 5509
   ctgggcagaa cgtaagccgc gagagcgcca acaaccgctt cttggtcgaa ggcagcaagc 5569
   gcgatgaatg tcttactacg gagcaagttc ccgaggtaat cggagtccgg ctgatgttgg 5629
   gagtaggtgg ctacgtctcc gaactcacga ccgaaaagat caagagcagc ccgcatggat 5689
   ttgacttggt cagggccgag cctacatgtg cgaatgatgc ccatacttga gccacctaac 5749
   tttgttttag ggcgactgcc ctgctgcgta acatcgttgc tgctgcgtaa catcgttgct 5809
   gctccataac atcaaacatc gacccacggc gtaacgcgct tgctgcttgg atgcccgagg 5869
   catagactgt acaaaaaaac agtcataaca agccatgaaa accgccactg cgccgttacc 5929
   accgctgcgt tcggtcaagg ttctggacca gttgcgtgag cgcatacgct acttgcatta 5989
   cagtttacga accgaacagg cttatgtcaa ctgggttcgt gccttcatcc gtttccacgg 6049
   tgtgcgtcac ccggcaacct tgggcagcag cgaagtcgag gcatttctgt cctggctggc 6109
   gaacgagcgc aaggtttcgg tctccacgca tcgtcaggca ttggcggcct tgctgttctt 6169
   ctacggcaag gtgctgtgca cggatctgcc ctggcttcag gagatcggta gacctcggcc 6229
   gtcgcggcgc ttgccggtgg tgctgacccc ggatgaagtg gttcgcatcc tcggttttct 6289
   ggaaggcgag catcgtttgt tcgcccagga ctctagctat agttctagtg gttggcctac 6349
   gtacccgtag tggctatggc agggcttgcc gccccgacgt tggctgcgag ccctgggcct 6409
   tcacccgaac ttgggggttg gggtggggaa aaggaagaaa cgcgggcgta ttggtcccaa 6469
   tggggtctcg gtggggtatc gacagagtgc cagccctggg accgaacccc gcgtttatga 6529
   acaaacgacc caacacccgt gcgttttatt ctgtcttttt attgccgtca tagcgcgggt 6589
   tccttccggt attgtctcct tccgtgtttc agttagcctc ccccatctcc cggtaccgca 6649
   tgcctcgaga ctgcaggctc tagattcgaa agcggccgcg actagtgagc tcgtcgacgt 6709
   aggcctttga attccggatc ctcactcaag gtcctcatca gagacggtcc tgatccagcg 6769
   gcccagccga ccagggggtc tctgtgttgg agcattgggt tttggcttgg gctttggtag 6829
   agcccgcctg ggattgcgat gcttcatctc catcgcagtc aagagcagat ctttcatctg 6889
   ttcttggttt gggccacgtc catggttgat ttcatagact ttggcaactt cgtctatgaa 6949
   agcttggggt ggctctgcct gtcctggagc cccgtagatc gacgtagctg cccttaggat 7009
   ttgttcttct gatgccaacc ggctcttctc tgcatgcacg tagtctagat agtcctcgtt 7069
   tgggtccggt atttctcgtt tgttctgcca gtactttacc tggcctgggc ttggccctcg 7129
   gtgcccattg agtgctaccc attctggtgt tgcaaagtag atgcccatgg tctccatctt 7189
   ctttgagatc cgtgtgtctt tttccctctg tgcttcctct ggtgtggggc cccgagcctc 7249
   cactccgtag cctgctgtcc cgtacttggc cctttgcgac ttgctgcctg cttgtggtgc 7309
   gtttgcaaga aaatttcgca tccgatgggc gttcgggtcg ctgagtgcga agttggccat 7369
   gtcagtcaca atcccattct cttccagcca catgaacaca ctgagtgcag attggaatag 7429
   tgggtccacg ttggctgctg cttccattgc tctgacggca ctctcgagtt cgggggtctc 7489
   tttgaactct gatgcagcca tggcgccctg aaaatacagg ttttcggtcg ttgggatatc 7549
   gtaatcgtga tggtgatggt gatggtagta cgacatggtt tcggac 7595
<210> 4
   <211> 333
   <212> PRT
   <213> Artificial sequence
<220>
   <223> pVP2-his-VP3 protein
<400> 4
   Met Thr Asn Leu Ser Asp Gln Thr Gln Gln Ile Val Pro Phe Ile Arg
   1 5 10 15
   Ser Leu Leu Met Pro Thr Thr Gly Pro Ala Ser Ile Pro Asp Asp Thr
   20 25 30
   Leu Glu Lys His Thr Leu Arg Ser Glu Thr Ser Thr Tyr Asn Leu Thr
   35 40 45
   Val Gly Asp Thr Gly Ser Gly Leu Ile Val Phe Phe Pro Gly Phe Pro
   50 55 60
   Gly Ser Ile Val Gly Ala His Tyr Thr Leu Gln Gly Asn Gly Asn Tyr
   65 70 75 80
   Lys Phe Asp Gln Met Leu Leu Thr Ala Gln Asn Leu Pro Ala Ser Tyr
   85 90 95
   Asn Tyr Cys Arg Leu Val Ser Arg Ser Leu Thr Val Arg Ser Ser Thr
   100 105 110
   Leu Pro Gly Gly Val Tyr Ala Leu Asn Gly Thr Ile Asn Ala Val Thr
   115 120 125
   Phe Gln Gly Ser Leu Ser Glu Leu Thr Asp Val Ser Tyr Asn Gly Leu
   130 135 140
   Met Ser Ala Thr Ala Asn Ile Asn Asp Lys Ile Gly Asn Val Leu Val
   145 150 155 160
   Gly Glu Gly Val Thr Val Leu Ser Leu Pro Thr Ser Tyr Asp Leu Gly
   165 170 175
   Tyr Val Arg Leu Gly Asp Pro Ile Pro Ala Ile Gly Leu Asp Pro Lys
   180 185 190
   Met Val Ala Thr Cys Asp Ser Ser Asp Arg Pro Arg Val Tyr Thr Ile
   195 200 205
   Thr Ala Ala Asp Asp Tyr Gln Phe Ser Ser Gln Tyr Gln Pro Gly Gly
       210                 215                 220
   Val Thr Ile Thr Leu Phe Ser Ala Asn Ile Asp Ala Ile Thr Ser Leu
   225                 230                 235                 240
   Ser Val Gly Gly Glu Leu Val Phe Arg Thr Ser Val His Gly Leu Val
                   245                 250                 255
   Leu Gly Ala Thr Ile Tyr Leu Ile Gly Phe Asp Gly Thr Thr Val Ile
               260                 265                 270
   Thr Arg Ala Val Ala Ala Asn Asn Gly Leu Thr Thr Gly Thr Asp Asn
           275                 280                 285
   Leu Met Pro Phe Asn Leu Val Ile Pro Thr Asn Glu Ile Thr Gln Pro
       290                 295                 300
   Ile Thr Ser Ile Lys Leu Glu Ile Val Thr Ser Lys Ser Gly Gly Gln
   305                 310                 315                 320
   Ala Gly Asp Gln Met Ser Trp Ser Ala Arg Gly Ser Leu
                   325                 330
<210> 5
   <211> 35
   <212> DNA
   <213> Artificial sequence
<220> Synthetic DNA
   <223> Oligo III primer
<400> 5
   gcgcagatct atgacaaacc tgtcagatca aaccc 35
<210> 6
   <211> 34
   <212> DNA
   <213> Artificial sequence
<220> Synthetic DNA
   <223> Oligo IV primer
<400> 6
   gcgcaagctt aggcgagagt cagctgcctt atgc 34
<210> 7
   <211> 33
   <212> DNA
   <213> Artificial sequence
<220> Synthetic DNA
   <223> Oligo V primer
<400> 7
   gcgcgaattc gatggcatca gagttcaaag aga 33
<210> 8
   <211> 32
   <212> DNA
   <213> Artificial sequence
<220> Synthetic DNA
   <223> Oligo VI primer
<400> 8
   cgcggatccc tcaaggtcct catcagagac gg 32
<210> 9
   <211> 9600
   <212> DNA
   <213> Artificial sequence
<223> Plasmid pESCURA/pVP2-VP3-GFP
<221> promoter
   <222> (5649).. (5859)
   <223> Promoter GAL 1 (pVP2)
<221> promoter
   <222> (7402)..(8080)
   <223> Promoter GAL 2 (VP3-GFP)
<221> CDS
   <222> (8086)..(9597)
   <223> VP3-GFP ORF
<400> 9
   ggccgcacta gtatcgatgg attacaagga tgacgacgat aagatctgag ctcttaatta 60
   acaattcttc gccagaggtt tggtcaagtc tccaatcaag gttgtcggct tgtctacctt 120
   gccagaaatt tacgaaaaga tggaaaaggg tcaaatcgtt ggtagatacg ttgttgacac 180
   ttctaaataa gcgaatttct tatgatttat gatttttatt attaaataag ttataaaaaa 240
   aataagtgta tacaaatttt aaagtgactc ttaggtttta aaacgaaaat tcttattctt 300
   gagtaactct ttcctgtagg tcaggttgct ttctcaggta tagcatgagg tcgctccaat 360
   tcagctgcat taatgaatcg gccaacgcgc ggggagaggc ggtttgcgta ttgggcgctc 420
   ttccgcttcc tcgctcactg actcgctgcg ctcggtcgtt cggctgcggc gagcggtatc 480
   agctcactca aaggcggtaa tacggttatc cacagaatca ggggataacg caggaaagaa 540
   catgtgagca aaaggccagc aaaaggccag gaaccgtaaa aaggccgcgt tgctggcgtt 600
   tttccatagg ctccgccccc ctgacgagca tcacaaaaat cgacgctcaa gtcagaggtg 660
   gcgaaacccg acaggactat aaagatacca ggcgtttccc cctggaagct ccctcgtgcg 720
   ctctcctgtt ccgaccctgc cgcttaccgg atacctgtcc gcctttctcc cttcgggaag 780
   cgtggcgctt tctcatagct cacgctgtag gtatctcagt tcggtgtagg tcgttcgctc 840
   caagctgggc tgtgtgcacg aaccccccgt tcagcccgac cgctgcgcct tatccggtaa 900
   ctatcgtctt gagtccaacc cggtaagaca cgacttatcg ccactggcag cagccactgg 960
   taacaggatt agcagagcga ggtatgtagg cggtgctaca gagttcttga agtggtggcc 1020
   taactacggc tacactagaa ggacagtatt tggtatctgc gctctgctga agccagttac 1080
   cttcggaaaa agagttggta gctcttgatc cggcaaacaa accaccgctg gtagcggtgg 1140
   tttttttgtt tgcaagcagc agattacgcg cagaaaaaaa ggatctcaag aagatccttt 1200
   gatcttttct acggggtctg acgctcagtg gaacgaaaac tcacgttaag ggattttggt 1260
   catgagatta tcaaaaagga tcttcaccta gatcctttta aattaaaaat gaagttttaa 1320
   atcaatctaa agtatatatg agtaaacttg gtctgacagt taccaatgct taatcagtga 1380
   ggcacctatc tcagcgatct gtctatttcg ttcatccata gttgcctgac tccccgtcgt 1440
   gtagataact acgatacggg agggcttacc atctggcccc agtgctgcaa tgataccgcg 1500
   agacccacgc tcaccggctc cagatttatc agcaataaac cagccagccg gaagggccga 1560
   gcgcagaagt ggtcctgcaa ctttatccgc ctccatccag tctattaatt gttgccggga 1620
   agctagagta agtagttcgc cagttaatag tttgcgcaac gttgttgcca ttgctacagg 1680
   catcgtggtg tcacgctcgt cgtttggtat ggcttcattc agctccggtt cccaacgatc 1740
   aaggcgagtt acatgatccc ccatgttgtg caaaaaagcg gttagctcct tcggtcctcc 1800
   gatcgttgtc agaagtaagt tggccgcagt gttatcactc atggttatgg cagcactgca 1860
   taattctctt actgtcatgc catccgtaag atgcttttct gtgactggtg agtactcaac 1920
   caagtcattc tgagaatagt gtatgcggcg accgagttgc tcttgcccgg cgtcaatacg 1980
   ggataatacc gcgccacata gcagaacttt aaaagtgctc atcattggaa aacgttcttc 2040
   ggggcgaaaa ctctcaagga tcttaccgct gttgagatcc agttcgatgt aacccactcg 2100
   tgcacccaac tgatcttcag catcttttac tttcaccagc gtttctgggt gagcaaaaac 2160
   aggaaggcaa aatgccgcaa aaaagggaat aagggcgaca cggaaatgtt gaatactcat 2220
   actcttcctt tttcaatatt attgaagcat ttatcagggt tattgtctca tgagcggata 2280
   catatttgaa tgtatttaga aaaataaaca aataggggtt ccgcgcacat ttccccgaaa 2340
   agtgccacct gaacgaagca tctgtgcttc attttgtaga acaaaaatgc aacgcgagag 2400
   cgctaatttt tcaaacaaag aatctgagct gcatttttac agaacagaaa tgcaacgcga 2460
   aagcgctatt ttaccaacga agaatctgtg cttcattttt gtaaaacaaa aatgcaacgc 2520
   gagagcgcta atttttcaaa caaagaatct gagctgcatt tttacagaac agaaatgcaa 2580
   cgcgagagcg ctattttacc aacaaagaat ctatacttct tttttgttct acaaaaatgc 2640
   atcccgagag cgctattttt ctaacaaagc atcttagatt actttttttc tcctttgtgc 2700
   gctctataat gcagtctctt gataactttt tgcactgtag gtccgttaag gttagaagaa 2760
   ggctactttg gtgtctattt tctcttccat aaaaaaagcc tgactccact tcccgcgttt 2820
   actgattact agcgaagctg cgggtgcatt ttttcaagat aaaggcatcc ccgattatat 2880
   tctataccga tgtggattgc gcatactttg tgaacagaaa gtgatagcgt tgatgattct 2940
   tcattggtca gaaaattatg aacggtttct tctattttgt ctctatatac tacgtatagg 3000
   aaatgtttac attttcgtat tgttttcgat tcactctatg aatagttctt actacaattt 3060
   ttttgtctaa agagtaatac tagagataaa cataaaaaat gtagaggtcg agtttagatg 3120
   caagttcaag gagcgaaagg tggatgggta ggttatatag ggatatagca cagagatata 3180
   tagcaaagag atacttttga gcaatgtttg tggaagcggt attcgcaata ttttagtagc 3240
   tcgttacagt ccggtgcgtt tttggttttt tgaaagtgcg tcttcagagc gcttttggtt 3300
   ttcaaaagcg ctctgaagtt cctatacttt ctagagaata ggaacttcgg aataggaact 3360
   tcaaagcgtt tccgaaaacg agcgcttccg aaaatgcaac gcgagctgcg cacatacagc 3420
   tcactgttca cgtcgcacct atatctgcgt gttgcctgta tatatatata catgagaaga 3480
   acggcatagt gcgtgtttat gcttaaatgc gtacttatat gcgtctattt atgtaggatg 3540
   aaaggtagtc tagtacctcc tgtgatatta tcccattcca tgcggggtat cgtatgcttc 3600
   cttcagcact accctttagc tgttctatat gctgccactc ctcaattgga ttagtctcat 3660
   ccttcaatgc tatcatttcc tttgatattg gatcatacta agaaaccatt attatcatga 3720
   cattaaccta taaaaatagg cgtatcacga ggccctttcg tctcgcgcgt ttcggtgatg 3780
   acggtgaaaa cctctgacac atgcagctcc cggagacggt cacagcttgt ctgtaagcgg 3840
   atgccgggag cagacaagcc cgtcagggcg cgtcagcggg tgttggcggg tgtcggggct 3900
   ggcttaacta tgcggcatca gagcagattg tactgagagt gcaccatacc acagcttttc 3960
   aattcaattc atcatttttt ttttattctt ttttttgatt tcggtttctt tgaaattttt 4020
   ttgattcggt aatctccgaa cagaaggaag aacgaaggaa ggagcacaga cttagattgg 4080
   tatatatacg catatgtagt gttgaagaaa catgaaattg cccagtattc ttaacccaac 4140
   tgcacagaac aaaaacctgc aggaaacgaa gataaatcat gtcgaaagct acatataagg 4200
   aacgtgctgc tactcatcct agtcctgttg ctgccaagct atttaatatc atgcacgaaa 4260
   agcaaacaaa cttgtgtgct tcattggatg ttcgtaccac caaggaatta ctggagttag 4320
   ttgaagcatt aggtcccaaa atttgtttac taaaaacaca tgtggatatc ttgactgatt 4380
   tttccatgga gggcacagtt aagccgctaa aggcattatc cgccaagtac aattttttac 4440
   tcttcgaaga cagaaaattt gctgacattg gtaatacagt caaattgcag tactctgcgg 4500
   gtgtatacag aatagcagaa tgggcagaca ttacgaatgc acacggtgtg gtgggcccag 4560
   gtattgttag cggtttgaag caggcggcag aagaagtaac aaaggaacct agaggccttt 4620
   tgatgttagc agaattgtca tgcaagggct ccctatctac tggagaatat actaagggta 4680
   ctgttgacat tgcgaagagc gacaaagatt ttgttatcgg ctttattgct caaagagaca 4740
   tgggtggaag agatgaaggt tacgattggt tgattatgac acccggtgtg ggtttagatg 4800
   acaagggaga cgcattgggt caacagtata gaaccgtgga tgatgtggtc tctacaggat 4860
   ctgacattat tattgttgga agaggactat ttgcaaaggg aagggatgct aaggtagagg 4920
   gtgaacgtta cagaaaagca ggctgggaag catatttgag aagatgcggc cagcaaaact 4980
   aaaaaactgt attataagta aatgcatgta tactaaactc acaaattaga gcttcaattt 5040
   aattatatca gttattaccc tatgcggtgt gaaataccgc acagatgcgt aaggagaaaa 5100
   taccgcatca ggaaattgta aacgttaata ttttgttaaa attcgcgtta aatttttgtt 5160
   aaatcagctc attttttaac caataggccg aaatcggcaa aatcccttat aaatcaaaag 5220
   aatagaccga gatagggttg agtgttgttc cagtttggaa caagagtcca ctattaaaga 5280
   acgtggactc caacgtcaaa gggcgaaaaa ccgtctatca gggcgatggc ccactacgtg 5340
   aaccatcacc ctaatcaagt tttttggggt cgaggtgccg taaagcacta aatcggaacc 5400
   ctaaagggag cccccgattt agagcttgac ggggaaagcc ggcgaacgtg gcgagaaagg 5460
   aagggaagaa agcgaaagga gcgggcgcta gggcgctggc aagtgtagcg gtcacgctgc 5520
   gcgtaaccac cacacccgcc gcgcttaatg cgccgctaca gggcgcgtcg cgccattcgc 5580
   cattcaggct gcgcaactgt tgggaagggc gatcggtgcg ggcctcttcg ctattacgcc 5640
   agctggatct tcgagcgtcc caaaaccttc tcaagcaagg ttttcagtat aatgttacat 5700
   gcgtacacgc gtctgtacag aaaaaaaaga aaaatttgaa atataaataa cgttcttaat 5760
   actaacataa ctataaaaaa ataaataggg acctagactt caggttgtct aactccttcc 5820
   ttttcggtta gagcggatct tagctagccg cggtaccaag cttaggcgag agtcagctgc 5880
   cttatgcggc ctgaggcagc tcttgctttt cctgacgcgg ctcgagcagt tcctgaagcg 5940
   gcctgggcct catcgcccag caggtagtct acaccttccc caattgcatg ggctagggga 6000
   gcggcaggtg ggaacaatgt ggagaccacc ggcacagcta tcctccttat ggcccggatt 6060
   atgtctttga agccgaatgc tcctgcaatc ttcaggggag agttgaggtc ggccacctcc 6120
   atgaagtatt cacgaaagtc agtgtactcc cttgttggcc agacggtctt gatgccaaga 6180
   cggtccctct cactcagtat caattttgtg tagttcatgg ctcctgggtc aaatcggccg 6240
   tattctgtaa ccaggttctt tgctagttca ggatttggga tcagctcgaa gttgctcacc 6300
   ccagcgaccg taacgacgga tcctgttgcc actctttcgt aggccactag cgtgacggga 6360
   cggagggccc ctggatagtt gccaccatgg atcgtcactg ctaggctccc tcttgccgac 6420
   catgacatct gatcccctgc ctgaccacca cttttggagg tcactatctc cagtttgatg 6480
   gatgtgattg gctgggttat ctcgtttgtt ggaatcacaa gattgaatgg cataaggttg 6540
   tcggtgccgg tcgtcagccc attgtttgcg gccacagccc tggtgattac cgttgtccca 6600
   tcaaagccta tgaggtagat ggtggcgccc agtacaaggc cgtggacgct tgttcgaaac 6660
   acgagctctc ccccaacgct gaggcttgtg atggcatcaa tgttggctga gaacagtgtg 6720
   attgttaccc cacctggttg gtactgtgat gagaattggt aatcatcggc tgcagttatg 6780
   gtgtagactc tgggcctgtc actgctgtca catgtggcta ccatttttgg gtcaagccct 6840
   attgcgggaa tggggtcacc aagcctcaca tacccaagat catatgatgt gggtaagctg 6900
   aggacggtga ccccttcccc tactaggacg ttcccaattt tgtcgttgat gttggctgtt 6960
   gcagacatca acccattgta gctaacatct gtcagttcac tcaggcttcc ttggaaggtc 7020
   acggcgttta tggtgccgtt tagtgcataa acgccaccag gaagtgtgct tgacctcact 7080
   gtgagactcc gactcactag cctgcagtag ttgtaactgg ccggtaggtt ctgggcagtc 7140
   aggagcatct gatcgaactt gtagttccca ttgccctgca gtgtgtagtg agcacccaca 7200
   attgagccag ggaatccagg gaaaaagaca attagccctg accctgtgtc ccccacagtc 7260
   aaattgtagg tcgaggtctc tgacctgaga gtgtgcttct ccagggtgtc gtccggaatg 7320
   gacgccggtc cggttgttgg catcagaagg ctccgtatga acggaacaat ctgctgggtt 7380
   tgatctgaca ggtttgtcat agatccgggg ttttttctcc ttgacgttaa agtatagagg 7440
   tatattaaca attttttgtt gatactttta ttacatttga ataagaagta atacaaaccg 7500
   aaaatgttga aagtattagt taaagtggtt atgcagtttt tgcatttata tatctgttaa 7560
   tagatcaaaa atcatcgctt cgctgattaa ttaccccaga aataaggcta aaaaactaat 7620
   cgcattatca tcctatggtt gttaatttga ttcgttcatt tgaaggtttg tggggccagg 7680
   ttactgccaa tttttcctct tcataaccat aaaagctagt attgtagaat ctttattgtt 7740
   cggagcagtg cggcgcgagg cacatctgcg tttcaggaac gcgaccggtg aagacgagga 7800
   cgcacggagg agagtcttcc ttcggagggc tgtcacccgc tcggcggctt ctaatccgta 7860
   cttcaatata gcaatgagca gttaagcgta ttactgaaag ttccaaagag aaggtttttt 7920
   taggctaaga taatggggct ctttacattt ccacaacata taagtaagat tagatatgga 7980
   tatgtatatg gatatgtata tggtggtaat gccatgtaat atgattatta aacttctttg 8040
   cgtccatcca aaaaaaaagt aagaattttt gaaaattcga attcg atg gct gca tca   8097
                                                     Met Ala Ala Ser
                                                     1
   gag ttc aaa gag acc ccc gaa ctc gag agt gcc gtc aga gca atg gaa    8145
   Glu Phe Lys Glu Thr Pro Glu Leu Glu Ser Ala Val Arg Ala Met Glu
   5                   10                  15                  20
   gca gca gcc aac gtg gac cca cta ttc caa tct gca ctc agt gtg ttc    8193
   Ala Ala Ala Asn Val Asp Pro Leu Phe Gln Ser Ala Leu Ser Val Phe
                   25                  30                  35
   atg tgg ctg gaa gag aat ggg att gtg act gac atg gcc aac ttc gca    8241
   Met Trp Leu Glu Glu Asn Gly Ile Val Thr Asp Met Ala Asn Phe Ala
               40                  45                  50
   ctc agc gac ccg aac gcc cat cgg atg cga aat ttt ctt gca aac gca    8289
   Leu Ser Asp Pro Asn Ala His Arg Met Arg Asn Phe Leu Ala Asn Ala
           55                  60                  65
   cca caa gca ggc agc aag tcg caa agg gcc aag tac ggg aca gca ggc    8337
   Pro Gln Ala Gly Ser Lys Ser Gln Arg Ala Lys Tyr Gly Thr Ala Gly
       70                  75                  80
   tac gga gtg gag gct cgg ggc ccc aca cca gag gaa gca cag agg gaa    8385
   Tyr Gly Val Glu Ala Arg Gly Pro Thr Pro Glu Glu Ala Gln Arg Glu
   85                  90                  95                  100
   aaa gac aca cgg atc tca aag aag atg gag acc atg ggc atc tac ttt    8433
   Lys Asp Thr Arg Ile Ser Lys Lys Met Glu Thr Met Gly Ile Tyr Phe
                   105                 110                 115
   gca aca cca gaa tgg gta gca ctc aat ggg cac cga ggg cca agc cca    8481
   Ala Thr Pro Glu Trp Val Ala Leu Asn Gly His Arg Gly Pro Ser Pro
               120                 125                 130
   ggc cag gta aag tac tgg cag aac aaa cga gaa ata ccg gac cca aac    8529
   Gly Gln Val Lys Tyr Trp Gln Asn Lys Arg Glu Ile Pro Asp Pro Asn
           135                 140                 145
   gag gac tat cta gac tac gtg cat gca gag aag agc cgg ttg gca tca    8577
   Glu Asp Tyr Leu Asp Tyr Val His Ala Glu Lys Ser Arg Leu Ala Ser
       150                 155                 160
   gaa gaa caa atc cta agg gca gct acg tcg atc tac ggg gct cca gga    8625
   Glu Glu Gln Ile Leu Arg Ala Ala Thr Ser Ile Tyr Gly Ala Pro Gly
   165                 170                 175                 180
   cag gca gag cca ccc caa gct ttc ata gac gaa gtt gcc aaa gtc tat    8673
   Gln Ala Glu Pro Pro Gln Ala Phe Ile Asp Glu Val Ala Lys Val Tyr
                   185                 190                 195
   gaa atc aac cat gga cgt ggc cca aac caa gaa cag atg aaa gat ctg    8721
   Glu Ile Asn His Gly Arg Gly Pro Asn Gln Glu Gln Met Lys Asp Leu
               200                 205                 210
   ctc ttg act gcg atg gag atg aag cat cgc aat ccc agg cgg gct cta    8769
   Leu Leu Thr Ala Met Glu Met Lys His Arg Asn Pro Arg Arg Ala Leu
           215                 220                 225
   cca aag ccc aag cca aaa ccc aat gct cca aca cag aga ccc cct ggt      8817
   Pro Lys Pro Lys Pro Lys Pro Asn Ala Pro Thr Gln Arg Pro Pro Gly
       230                 235                 240
   cgg ctg ggc cgc tgg atc agg acc gtc tct gat gag gac ctt gag gga      8865
   Arg Leu Gly Arg Trp Ile Arg Thr Val Ser Asp Glu Asp Leu Glu Gly
   245                 250                 255                 260
   tcc atc gcc acc atg gtg agc aag ggc gag gag ctg ttc acc ggg gtg      8913
   Ser Ile Ala Thr Met Val Ser Lys Gly Glu Glu Leu Phe Thr Gly Val
                   265                 270                 275
   gtg ccc atc ctg gtc gag ctg gac ggc gac gta aac ggc cac aag ttc      8961
   Val Pro Ile Leu Val Glu Leu Asp Gly Asp Val Asn Gly His Lys Phe
               280                 285                 290
   agc gtg tcc ggc gag ggc gag ggc gat gcc acc tac ggc aag ctg acc      9009
   Ser Val Ser Gly Glu Gly Glu Gly Asp Ala Thr Tyr Gly Lys Leu Thr
           295                 300                 305
   ctg aag ttc atc tgc acc acc ggc aag ctg ccc gtg ccc tgg ccc acc      9057
   Leu Lys Phe Ile Cys Thr Thr Gly Lys Leu Pro Val Pro Trp Pro Thr
       310                 315                 320
   ctc gtg acc acc ctg acc tac ggc gtg cag tgc ttc agc cgc tac ccc      9105
   Leu Val Thr Thr Leu Thr Tyr Gly Val Gln Cys Phe Ser Arg Tyr Pro
   325                 330                 335                 340
   gac cac atg aag cag cac gac ttc ttc aag tcc gcc atg ccc gaa ggc      9153
   Asp His Met Lys Gln His Asp Phe Phe Lys Ser Ala Met Pro Glu Gly
                   345                 350                 355
   tac gtc cag gag cgc acc atc ttc ttc aag gac gac ggc aac tac aag      9201
   Tyr Val Gln Glu Arg Thr Ile Phe Phe Lys Asp Asp Gly Asn Tyr Lys
               360                 365                 370
   acc cgc gcc gag gtg aag ttc gag ggc gac acc ctg gtg aac cgc atc      9249
   Thr Arg Ala Glu Val Lys Phe Glu Gly Asp Thr Leu Val Asn Arg Ile
           375                 380                 385
   gag ctg aag ggc atc gac ttc aag gag gac ggc aac atc ctg ggg cac      9297
   Glu Leu Lys Gly Ile Asp Phe Lys Glu Asp Gly Asn Ile Leu Gly His
       390                 395                 400
   aag ctg gag tac aac tac aac agc cac aac gtc tat atc atg gcc gac      9345
   Lys Leu Glu Tyr Asn Tyr Asn Ser His Asn Val Tyr Ile Met Ala Asp
   405                 410                 415                 420
   aag cag aag aac ggc atc aag gtg aac ttc aag atc cgc cac aac atc      9393
   Lys Gln Lys Asn Gly Ile Lys Val Asn Phe Lys Ile Arg His Asn Ile
                   425                 430                 435
   gag gac ggc agc gtg cag ctc gcc gac cac tac cag cag aac acc ccc      9441
   Glu Asp Gly Ser Val Gln Leu Ala Asp His Tyr Gln Gln Asn Thr Pro
               440                 445                 450
   atc ggc gac ggc ccc gtg ctg ctg ccc gac aac cac tac ctg agc acc      9489
   Ile Gly Asp Gly Pro Val Leu Leu Pro Asp Asn His Tyr Leu Ser Thr
           455                 460                 465
   cag tcc gcc ctg agc aaa gac ccc aac gag aag cgc gat cac atg gtc      9537
   Gln Ser Ala Leu Ser Lys Asp Pro Asn Glu Lys Arg Asp His Met Val
       470                 475                 480
   ctg ctg gag ttc gtg acc gcc gcc ggg atc act ctc ggc atg gac gag      9585
   Leu Leu Glu Phe Val Thr Ala Ala Gly Ile Thr Leu Gly Met Asp Glu
   485                 490                 495                 500
   ctg tac aag taa agc                                                  9600
   Leu Tyr Lys
<210> 10
   <211> 503
   <212> PRT
   <213> Artificial sequence
<220>
   <223> pVP2-VP3-GFP protein
<400> 10
   Met Ala Ala Ser Glu Phe Lys Glu Thr Pro Glu Leu Glu Ser Ala Val
   1               5                   10                  15
   Arg Ala Met Glu Ala Ala Ala Asn Val Asp Pro Leu Phe Gln Ser Ala
               20                  25                  30
   Leu Ser Val Phe Met Trp Leu Glu Glu Asn Gly Ile Val Thr Asp Met
           35                  40                  45
   Ala Asn Phe Ala Leu Ser Asp Pro Asn Ala His Arg Met Arg Asn Phe
       50                  55                  60
   Leu Ala Asn Ala Pro Gln Ala Gly Ser Lys Ser Gln Arg Ala Lys Tyr
   65                  70                  75                  80
   Gly Thr Ala Gly Tyr Gly Val Glu Ala Arg Gly Pro Thr Pro Glu Glu
                   85                  90                  95
   Ala Gln Arg Glu Lys Asp Thr Arg Ile Ser Lys Lys Met Glu Thr Met
               100                 105                 110
   Gly Ile Tyr Phe Ala Thr Pro Glu Trp Val Ala Leu Asn Gly His Arg
           115                 120                 125
   Gly Pro Ser Pro Gly Gln Val Lys Tyr Trp Gln Asn Lys Arg Glu Ile
       130                 135                 140
   Pro Asp Pro Asn Glu Asp Tyr Leu Asp Tyr Val His Ala Glu Lys Ser
   145                 150                 155                 160
   Arg Leu Ala Ser Glu Glu Gln Ile Leu Arg Ala Ala Thr Ser Ile Tyr
                   165                 170                 175
   Gly Ala Pro Gly Gln Ala Glu Pro Pro Gln Ala Phe Ile Asp Glu Val
               180                 185                 190
   Ala Lys Val Tyr Glu Ile Asn His Gly Arg Gly Pro Asn Gln Glu Gln
           195                 200                 205
   Met Lys Asp Leu Leu Leu Thr Ala Met Glu Met Lys His Arg Asn Pro
       210                 215                 220
   Arg Arg Ala Leu Pro Lys Pro Lys Pro Lys Pro Asn Ala Pro Thr Gln
   225                 230                 235                 240
   Arg Pro Pro Gly Arg Leu Gly Arg Trp Ile Arg Thr Val Ser Asp Glu
                   245                 250                 255
   Asp Leu Glu Gly Ser Ile Ala Thr Met Val Ser Lys Gly Glu Glu Leu
               260                 265                 270
   Phe Thr Gly Val Val Pro Ile Leu Val Glu Leu Asp Gly Asp Val Asn
           275                 280                 285
   Gly His Lys Phe Ser Val Ser Gly Glu Gly Glu Gly Asp Ala Thr Tyr
       290                 295                 300
   Gly Lys Leu Thr Leu Lys Phe Ile Cys Thr Thr Gly Lys Leu Pro Val
   305                 310                 315                 320
   Pro Trp Pro Thr Leu Val Thr Thr Leu Thr Tyr Gly Val Gln Cys Phe
                   325                 330                 335
   Ser Arg Tyr Pro Asp His Met Lys Gln His Asp Phe Phe Lys Ser Ala
               340                 345                 350
   Met Pro Glu Gly Tyr Val Gln Glu Arg Thr Ile Phe Phe Lys Asp Asp
           355                 360                 365
   Gly Asn Tyr Lys Thr Arg Ala Glu Val Lys Phe Glu Gly Asp Thr Leu
       370                 375                 380
   Val Asn Arg Ile Glu Leu Lys Gly Ile Asp Phe Lys Glu Asp Gly Asn
   385                 390                 395                 400
   Ile Leu Gly His Lys Leu Glu Tyr Asn Tyr Asn Ser His Asn Val Tyr
                   405                 410                 415
   Ile Met Ala Asp Lys Gln Lys Asn Gly Ile Lys Val Asn Phe Lys Ile
               420                 425                 430
   Arg His Asn Ile Glu Asp Gly Ser Val Gln Leu Ala Asp His Tyr Gln
           435                 440                 445
   Gln Asn Thr Pro Ile Gly Asp Gly Pro Val Leu Leu Pro Asp Asn His
       450                 455                 460
   Tyr Leu Ser Thr Gln Ser Ala Leu Ser Lys Asp Pro Asn Glu Lys Arg
   465                 470                 475                 480
   Asp His Met Val Leu Leu Glu Phe Val Thr Ala Ala Gly Ile Thr Leu
                   485                 490                 495
   Gly Met Asp Glu Leu Tyr Lys
               500